(19) Europäisches Patentamt
European Patent Office
Office européen des brevets

(11) Veröffentlichungsnummer: **0 249 169 B1**

(12) # EUROPÄISCHE PATENTSCHRIFT

(45) Veröffentlichungstag der Patentschrift: **20.01.93**

(51) Int. Cl.$^5$: **C07K 7/06**, A61K 37/02

(21) Anmeldenummer: **87108184.0**

(22) Anmeldetag: **05.06.87**

(54) **Peptide mit Einfluss auf die Diurese und Natriurese, Verfahren zu ihrer Herstellung, diese enthaltende Mittel und ihre Verwendung.**

(30) Priorität: **11.06.86 DE 3619633**

(43) Veröffentlichungstag der Anmeldung:
**16.12.87 Patentblatt 87/51**

(45) Bekanntmachung des Hinweises auf die Patenterteilung:
**20.01.93 Patentblatt 93/03**

(84) Benannte Vertragsstaaten:
**AT BE CH DE ES FR GB GR IT LI LU NL SE**

(56) Entgegenhaltungen:
**EP-A- 0 140 731**
**WO-A-85/02850**

(73) Patentinhaber: **HOECHST AKTIENGESELLSCHAFT**
**Postfach 80 03 20**
**W-6230 Frankfurt am Main 80(DE)**

(72) Erfinder: **König, Wolfgang, Dr.**
**Eppsteiner Strasse 25**
**W-6238 Hofheim am Taunus(DE)**
Erfinder: **Breipohl, Gerhard, Dr.**
**Geisenheimer Strasse 95**
**W-6000 Frankfurt am Main(DE)**
Erfinder: **Geiger, Rolf, Prof. Dr.**
**Heinrich-Bleicher-Strasse 33**
**W-6000 Frankfurt am Main(DE)**
Erfinder: **Knolle, Jochen, Dr.**
**Höchster Strasse 21**
**W-6239 Kriftel(DE)**
Erfinder: **Hropot, Max, Dr.**
**Friedrich-Stolz-Strasse 13**
**W-6093 Flörsheim am Main(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services

## Beschreibung

Die Erfindung betrifft neue Peptide der Formel I,

$R^N$-L-N-$B^2$-$R^C$ (I)

in welcher

| | |
|---|---|
| $R^N$ | für einen Rest der Formel II steht; |

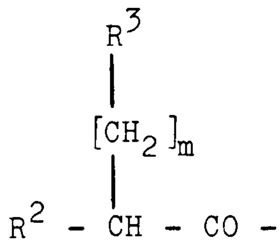

(II)

| | |
|---|---|
| $R^2$ | für Wasserstoff oder einen Rest der Formel R-$[A]_n$-NH- steht; |
| $R^3$ | Amino, Guanidino, ($C_1$-$C_3$)-Alkylamino oder Di-($C_1$-$C_3$)-alkylamino bedeutet; |
| m | eine ganze Zahl von 1-6 bedeutet; |
| A | für einen Rest der Formel -NH-$CR^4R^5$-CO- steht; |
| R | Wasserstoff, ($C_1$-$C_6$)-Alkanoyl, ($C_7$-$C_{11}$)-Aroyl, das im aromatischen Teil gegebenenfalls durch ($C_1$-$C_4$)-Alkyl, ($C_1$-$C_4$)-Alkoxy, ($C_1$-$C_4$)-Alkylthio, Halogen, Carbamoyl, ($C_1$-$C_4$)-Alkoxycarbonyl und/oder Sulfamoyl mono- oder disubstituiert oder durch Methylendioxy monosubstituiert ist, ($C_5$-$C_7$)-Cycloalkyl-($C_1$-$C_3$)-alkanoyl oder ($C_6$-$C_{14}$)-Aryl-($C_1$-$C_3$)-alkanoyl bedeutet, wobei in den Resten R $\neq$ Wasserstoff eine -$CH_2$-Gruppe gegebenenfalls durch -O- oder -S- ersetzt ist; |
| n | 0 oder 1 ist; |
| $R^4$ und $R^5$ | gleich oder verschieden sind und Wasserstoff, ($C_1$-$C_6$)-Alkyl oder ($C_7$-$C_{11}$)-Aralkanoyl bedeuten; |
| L | für den Rest einer lipophilen, neutralen $\alpha$-Aminosäure, vorzugsweise für Pro, D-Pro oder einen Rest der Formel -NH-CH($R^6$)-CO- steht; |
| $R^6$ | ($C_1$-$C_6$)-Alkyl, das gegebenenfalls durch Hydroxy, ($C_1$-$C_6$)-Alkoxy, ($C_1$-$C_6$)-Alkoxycarbonyl, Carbamoyl oder R-NH monosubstituiert ist, wobei R wie oben definiert ist, aber nicht Wasserstoff sein kann, oder ($C_7$-$C_{11}$)-Aralkyl, das am Aromaten gegebenenfalls durch ($C_1$-$C_6$)-Alkoxy monosubstituiert ist, oder Indol-3-ylmethyl bedeutet; |
| N | für den Rest einer neutralen $\alpha$-Aminosäure, vorzugsweise für Pro, D-Pro oder einen Rest der Formel -NH-CH($R^7$)-CO-steht; |
| $R^7$ | ($C_1$-$C_6$)-Alkyl, das gegebenenfalls durch Hydroxy, ($C_1$-$C_6$)-Alkoxy, ($C_1$-$C_6$)-Alkoxycarbonyl, Carbamoyl oder R-NH monosubstituiert ist, wobei R wie oben definiert ist, aber nicht Wasserstoff sein kann, oder ($C_7$-$C_{11}$)-Aralkyl, das am Aromaten gegebenenfalls durch ($C_1$-$C_6$)-Alkoxy monosubstituiert ist, oder Indol-3-ylmethyl bedeutet; |
| $B^2$ | für den Rest einer basischen $\alpha$-Aminosäure, vorzugsweise für einen Rest der Formel III |

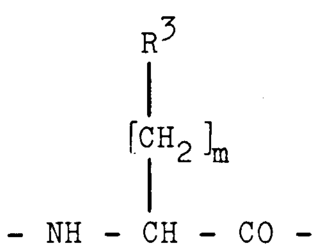

(III)

worin $R^3$ und m wie oben definiert sind, steht;

$R^C$ für eine Rest der Formel $-NR^9-CH(R^8)-(CO)_p-R^1$ steht;

$R^8$ eine lipophile Seitenkette bedeutet, die vorzugsweise wie $R^7$ definiert ist, wobei vorhandene CH-, $CH_2$- oder $CH_3$-Reste in β-Stellung bezüglich -NH- gegebenenfalls monohydroxyliert sind, und

$R^9$ Wasserstoff bedeutet;

oder

$R^8$ und $R^9$ zusammen $-[CH_2]_3-$ oder $-[CH_2]_4-$ bedeuten;

p 0 oder 1 ist;

$R^1$ im Falle von p = 0 für Wasserstoff, Hydroxy oder $(C_1-C_6)$-Alkoxy steht;

$R^1$ im Falle von p = 1 für $OR^{10}$ oder $NR^{10}R^{11}$ steht;

und

$R^{10}$ und $R^{11}$ gleich oder verschieden sind und Wasserstoff, $(C_1-C_6)$-Alkyl oder $(C_7-C_{11})$-Aralkyl bedeuten; oder $NR^{10}R^{11}$ für Pyrrolidino, Piperidino oder Morpholino steht;

oder

p = 1 ist,

R und $R^1$ gemeinsam eine Bindung bedeuten und die übrigen Reste wie oben definiert sind,

sowie deren physiologisch verträglichen Salze.

Als Salze kommen insbesondere Salze mit anorganischen oder organischen Säuren wie z.B. HCl, HBr, $H_2SO_4$, $H_3PO_4$, Maleinsäure, Fumarsäure, Citronensäure, Weinsäure, Essigsäure in Frage. Die Chiralitätszentren in den neuen Peptiden können jeweils die R-, S- oder R,S-Konfiguration besitzen.

Alkyl kann geradkettig oder verzweigt sein. Entsprechendes gilt für davon abgeleitete Reste, wie z.B. Alkoxy, Alkylthio, Alkylamino, Dialkylamino, Alkanoyl und Aralkyl.

Unter Cycloalkyl werden auch alkylsubstituierte Reste, wie z.B. 4-Methylcyclohexyl oder 2,3-Dimethylcyclopentyl verstanden.

$(C_6-C_{14})$-Aryl ist beispielsweise Phenyl, Naphthyl, Biphenylyl oder Fluorenyl; bevorzugt ist Phenyl. Entsprechendes gilt für davon abgeleitete Reste, wie z.B. Aryloxy, Aroyl, Aralkyl und Aralkyloxy. Bevorzugte Aralkylreste sind Benzyl und Phenethyl. Halogen bedeutet Fluor, Chlor, Brom oder Jod, vorzugsweise Chlor oder Brom.

Bevorzugt sind solche Reste $R^N$, L, N, $B^2$ und $R^C$, die sich von natürlichen vorkommenden Aminosäuren (s. z.B. Schröder , Lübke, The Peptides, Volume I, New York 1965, Seiten 137-270), deren Antipoden oder deren einfachen Metaboliten ableiten.

Solche Verbindungen der Formel I sind bevorzugt, worin

L für den Rest von Isoleucin, Valin, Threonin, Serin, O-$(C_1-C_9)$-Alkylthreonin, O-$(C_1-C_9)$-Alkylserin, Leucin, Prolin oder des ω-$(C_1-C_6)$-Alkyl- vorzugsweise tert. Butylesters von Glutaminsäure oder Asparaginsäure steht;

N für den Rest von Valin, Isoleucin, Leucin, Phenylalanin, Tryptophan, gegebenenfalls O-$(C_1-C_6)$-alkyliertem Tyrosin, Glutamin, Asparagin, Glutaminsäure-γ-$(C_1-C_6)$-alkylester oder Asparaginsäure-β-$(C_1-C_6)$-alkylester oder ε-Acyl-lysin steht,

und

$B^2$ Arg, D-Arg, Lys oder D-Lys bedeutet.

Die neuen Peptide sind entweder offenkettig oder über eine Peptidbindung (R, $R^1$ = Peptidbindung) cyclisch verknüpft.

Die Erfindung betrifft weiterhin ein Verfahren zur Herstellung von Verbindungen der Formel I, das dadurch gekennzeichnet ist, daß man ein Fragment mit endständiger Carboxylgruppe oder dessen reaktives Derivat mit einem entsprechenden Fragment mit freier Aminogruppe kuppelt, gegebenenfalls zum Schutz weiterer funktioneller Gruppen (eine) temporär eingeführte Schutzgruppe(n) abspaltet und die so erhaltene Verbindung gegebenenfalls in ihr physiologisch verträgliches Salz überführt.

Fragmente einer Verbindung der Formel I mit einer endständigen Carboxylgruppe besitzen die nachstehenden Formeln IVa-IVf

R-OH (IVa)

R-A-OH (IVb)

$R^N$-OH (IVc)

$R^N$-L-OH (IVd)

$R^N$-L-N-OH (IVe)

$R^N$-L-N-$B^2$-OH (IVf)

Fragmente einer Verbindung der Formel I mit einer endständigen Aminogruppe besitzen die nachstehenden Formeln Va-Vf

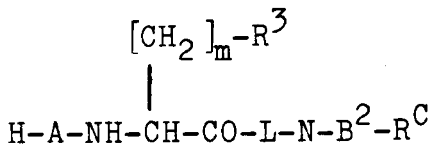

(Va)

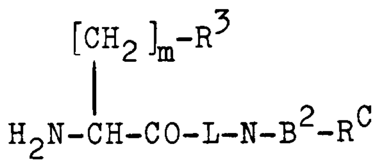

(Vb)

$H-L-N-B^2-R^6$ (Vc)
$H-N-B^2-R^6$ (Vd)
$H-B^2-R^C$ (Ve)
$H-R^C$ (Vf)

Die Synthese der erfindungsgemäßen Verbindungen folgt den bekannten Methoden der Peptidchemie, wie sie z.B. in Houben-Weyl, Methoden der Organischen Chemie, Band 15 (Stuttgart, Thieme, 1974) ausführlich beschrieben sind. Die Peptide werden vorzugsweise schrittweise beginnend mit der C-terminalen Aminosäure oder durch Fragmentkupplung nach dem Schema 2 + 3 oder 3 + 2 hergestellt. Bei der Fragmentkupplung hat man darauf zu achten, möglichst racemisierungsarme Kupplungsmethoden zu verwenden. In den hier vorliegenden Beispielen wurde zur Fragmentkupplung vor allem die DCC/HOObt-Methode eingesetzt, die nach den bisherigen Erfahrungen mit die geringste Racemisierung während der Peptidsynthese liefert. Weiterhin geeignet sind die Aktivestermethode mit N-Hydroxy-succinimid als Esterkomponente, die Kupplung mit Hilfe von Propanphosphonsäureanhydrid und die Gemischt-Anhydrid-Methode mit Pivaloylchlorid.

Die Herstellung von Cyclopeptiden der Formel I kann beispielsweise nach der in der EP-A2-135722 beschriebenen Verfahrensweise erfolgen.

Wie die nachstehenden Ergebnisse von in vivo-Tests zeigen, beeinflussen die erfindungsemäßen Verbindungen nachhaltig die Diurese, Natriurese und Kaliurese.

A. Diuretische, natriuretische und kaliuretische Wirkung der erfindungsgemäßen Peptide nach Versuchsanordnung 1 (Dosis 1 mg/kg Ratte, Sammeldauer: 90 min.)

| | Urin (ml·kg$^{-1}$) | $Na^+$ (µmol·kg$^{-1}$) | $K^+$ (µmol·kg$^{-1}$) |
|---|---|---|---|
| Kontrolle | 1,267 | 65,2 | 29,5 |
| H-Arg-Ile-Asp(OtBu)-Arg-Ile-OH | 2,49 | 249,1 | 25,9 |
| Phenoxyacetyl-Arg-Ile-Asp(OtBu)-Arg-Ile-OH | 0,66 | 117,7 | 31,7 |
| H-Arg-Ile-Asp(OtBu)-Arg-Ile-ol | 1,56 | 155,4 | 59,4 |
| H-Arg-Ile-Asp(OtBu)-Arg-Leu-ol | 1,55 | 180,8 | 64,2 |
| H-Arg-Ile-Asp(OtBu)-Arg-Val-$NH_2$ | 1,95 | 184,7 | 89,2 |
| H-Arg-Ile-Asp(OtBu)-Arg-isobutylamid | 1,43 | 131,6 | 51,3 |
| H-Arg-Ile-Asp(OtBu)-Arg-Trp-$NH_2$ | 1,97 | 225,0 | 33,0 |
| H-Arg-Ile-Asp(OtBu)-Arg-Ile-$NH_2$ | 1,34 | 159,8 | 35,4 |
| Z-Arg-Ile-Asp(OtBu)-Arg-Ile-ol (1 µg/kg) | 0,79 | 118,4 | 27,8 |
| Z-Arg-Ile-Asp(OtBu)-Arg-Ile-$NH_2$ | 1,51 | 263,2 | 63,3 |

(Dosis 1 mg/kg Ratte, Sammeldauer: 90 min.)

| | Urin (ml• kg$^{-1}$) | $Na^+$ (µmol• kg$^{-1}$) | $K^+$ (µmol• kg$^{-1}$) |
|---|---|---|---|
| H-Arg-Pro-Val-Lys-Val-OH | 0,91 | 155,7 | 28,2 |
| H-Arg-Ile-Ser(tBu)-Arg-Ile-OH | 1,19 | 74,5 | 28,4 |
| H-Arg-Ile-D-Ser(tBu)-Arg-Ile-OH | 0,95 | 87,0 | 44,3 |
| H-Arg-Ile-Leu-Arg-Ile-OH | 2,03 | 149,8 | 50,1 |
| H-Arg-Ile-Phe-Arg-Ile-OH | 2,00 | 211,0 | 68,3 |
| H-Arg-Ile-Trp-Arg-Ile-OH | 0,67 | 112,8 | 57,5 |
| Z-Arg-Ile-Trp-Arg-Ile-OH | 1,64 | 208,6 | 37,1 |
| Z-Arg-Ile-Trp-Arg-Ile-OMe | 1,16 | 152,5 | 46,2 |
| cyclo-(Gly-Arg-Ile-Phe-Arg-Ile) | 1,58 | 196,2 | 57,7 |
| H-Arg-Val-Tyr(tBu)-Arg-Pro-OtBu | 0,28 | 59,2 | 21,9 |

Versuchsanordnung 1: Die Ratten wurden mit i.p. 0,1 ml 10 %igem Thiobutabarbital-Natrium/100 g Körpergewicht narkotisiert. Sie erhielten ab 60 min. vor Substanzgabe eine NaCl-Infusion, die während des Versuchs fortgesetzt wurde (2,5 ml einer 0,9 %igen NaCl-Lösung•$h^{-1}$•0,1 $kg^{-1}$ Körpergewicht). Die Prüfsubstanz wurde als i.v. Bolus in 0,5 ml einer 0,9 %igen Lösung/100 g Körpergewicht appliziert.

B. Diuretische, natriuretische und kaliuretische Wirkung der erfindungsgemäßen Peptide nach Versuchsanordnung 2 (Dosis: 1 mg/kg Ratte, Sammeldauer: 120 min.)

| | Urin (ml• kg$^{-1}$) | $Na^+$ (µmol• kg$^{-1}$) | $K^+$ (µmol• kg$^{-1}$) |
|---|---|---|---|
| Kontrolle: | 0,520 | 31,5 | 25,8 |
| Z-Arg-Ile-Leu-D-Arg-Ile-OBzl | 0,52 | 66,9 | 36,2 |
| Z-D-Arg-Ile-Leu-Arg-Ile-OBzl | 0,59 | 86,3 | 38,1 |
| Z-Arg-Ile-D-Leu-Arg-D-Val-OBzl | 0,48 | 79,0 | 43,8 |

| | | | |
|---|---|---|---|
| Z-Arg-Ile-Leu-Arg-D-Val-OBzl | 0,43 | 88,6 | 36,7 |
| H-D-Arg-Ile-D-Leu-Arg-Ile-OH | 1,13 | 115,9 | 49,7 |
| H-Arg-Ile-Glu(OtBu)-Arg-Ile-$NH_2$ | 1,04 | 95,6 | 67,3 |
| ε-Amino-hexanoyl-Ile-Asp(OtBu)-Arg-Ile-$NH_2$ | 0,82 | 54,9 | 38,0 |
| Z-Arg-Ile-Glu(OtBu)-Arg-Ile-$NH_2$ | 0,47 | 91,5 | 36,1 |
| Z-D-Arg-Ile-Leu-D-Arg-Ile-OBzl | 0,29 | 48,4 | 30,1 |
| Z-D-Arg-Ile-D-Leu-Arg-Ile-OBzl | 0,19 | 37,3 | 20,4 |
| Z-Arg-D-Val-Leu-Arg-D-Val-OBzl | 0,27 | 55,1 | 38,3 |
| H-Arg-Leu-Gln-Arg-Leu-OH | 0,25 | 30,2 | 23,7 |
| H-Lys-Ile-Asp(OtBu)-Arg-Ile-$NH_2$ | 0,08 | 10,6 | 22,1 |
| H-Arg-D-Val-Leu-Arg-Ile-OH | 0,57 | 83,77 | 34,9 |
| H-D-Arg-D-allo-Ile-D-Leu-Arg-Ile-OH | 0,87 | 95,1 | 35,5 |
| H-Arg-Ile-Leu-D-Arg-Ile-OH | 0,78 | 93,7 | 40,8 |
| H-D-Arg-Ile-Leu-D-Arg-Ile-OH | 0,28 | 61,4 | 33,9 |
| H-D-Arg-Ile-Leu-Arg-Ile-OH | 0,22 | 55,3 | 33,6 |
| H-Arg-Ile-Leu-Arg-D-Val-OH | 0,56 | 68,8 | 38,9 |
| cyclo-(Gly-Arg-Ile-Leu-Arg-Ile) (mit 0,5 mg/kg) | 0,69 | 65,5 | 26,6 |
| H-Arg-Ile-D-Leu-Arg-D-Val-OH | 0,31 | 68,4 | 32,8 |
| Z-Arg-Ile-Ser(tBu)-Arg-Pro-OtBu | 0,44 | 88,9 | 37,8 |
| H-Arg-Ile-Ser(tBu)-Arg-Pro-OtBu | 0,63 | 136,8 | 31,5 |
| H-Arg-Trp-Asp(OtBu)-Arg-Phe-$NH_2$ | 0,66 | 148,7 | 37,1 |
| Fmoc-Arg-Pro-Cys(StBu)-Arg-Phe-OtBu | 0,65 | 143,0 | 16,5 |
| H-Arg-Pro-Cys(StBu)-Arg-Phe-OtBu | 0,32 | 87,5 | 11,1 |
| H-Lys-Phe-Leu-Lys-Phe-OH | 0,52 | 80,2 | 31,0 |
| H-Lys-Phe-Leu-Lys-Phe-$NH_2$ | 0,25 | 36,8 | 16,7 |
| cyclo-(D-Arg-Ile-D-Leu-Arg-Ile) (mit 1 µg/kg) | 1,05 | 128,2 | 36,6 |

Versuchsanordnung 2: Die Ratten wurden mit i.p. 0,1 ml 10 %igem Thiobutabarbital-Natrium/100 g Körpergewicht narkotisiert. Sie erhielten ab 60 min vor Substanzgabe bis zur Substanzgabe eine NaCl-Infusion (2,5 ml einer 0,85 %igen NaCl-Lösung•$h^{-1}$•0,1 $kg^{-1}$ Körpergewicht). Nach Substanzgabe wurde für eine Stunde die Infusionsgeschwindigkeit auf die Hälfte verringert (1,25 ml•$h^{-1}$0,1 $kg^{-1}$). Danach wurde bis zum Versuchsende die Infusionsgeschwindigkeit noch einmal auf die Hälfte reduziert (0,625 ml•$h^{-1}$•0,1 $kg^{-1}$). Die Prüfsubstanz wurde als i.v. Bolus in 0,1 ml einer 0,85 %igen NaCl-Lösung/100 g Körpergewicht appliziert.

Aus den Daten geht hervor, daß die erfindungsgemäßen Peptide sowohl diuretisch als auch natriuretisch wirken können. In vielen Beispielen findet man jedoch nur eine Natriurese, die manchmal sogar mit einer

Antidiurese einhergeht. Da die Kalium-Ausscheidung in manchen Fällen die Kontrolle nicht übersteigt, kann man aus dieser Substanzklasse kaliumzurückhaltende Natriuretica (z.B. H-Arg-Pro-Val-Lys-Val-OH), Antidiuretica mit natriuretischer Wirkung (z.B. Z-Arg-Ile-Asp(OtBu)-Arg-Ile-ol) oder Arzneimittel mit diuretischer und natriuretischer Wirkung (z.B. H-Arg-Ile-Asp(OtBu)-Arg-Ile-OH oder cyclo-(D-Arg-Ile-D-Leu-Arg-Ile) entwickeln. Aber auch Antidiuretica mit antinatriuretischer Wirkung (z.B. H-Arg-Pro-Cys(StBu)-Arg-Phe-OtBu) wurden in dieser Substanzklasse gefunden (z.B. H-Lys-Ile-Asp(OtBu)-Arg-Ile-$NH_2$).

Die Erfindung betrifft daher auch die Verwendung von Peptiden der Formel I als Arzneimittel mit regulatorischer Wirkung auf die Diurese, Natriurese bzw. Kaliurese, die bei Störungen des Wasser- bzw. Elektrolythaushaltes eingesetzt werden können, diese Peptide enthaltende pharmazeutische Mittel sowie Verfahren zur Herstellung der Mittel, die dadurch gekennzeichnet sind, daß man die Peptide zusammen mit einem Träger und gegebenenfalls weiteren Zusatz- und Hilfsstoffen in eine geeignete Darreichungsform bringt.

Die natriuretisch und diuretisch wirkenden Peptide lassen sich bei Hypertonie und Wassersucht als Arzneimittel einsetzen. Die Verbindungen, die spezifisch natriuretisch wirken, dürften bei älteren hypertonen Patienten eingesetzt werden, bei denen eine Entwässerung vermieden werden soll. Die antidiuretisch wirkenden Peptide sind bei Polyurie oder Diabetes insipidus angebracht.

Die Peptide können einzeln oder in Kombination parenteral (i.v., s.c., i.m.) in einem physiologisch verträglichen Medium verabreicht werden. Die zu verabreichende Dosis liegt in der Regel bei 1 $\mu$g - 5 mg/kg.

Zur parenteralen Applikation werden die aktiven Verbindungen oder deren physiologisch verträglichen Salze, gewünschtenfalls mit den dafür üblichen Substanzen wie Lösungsvermittler, Emulgatoren oder weitere Hilfsstoffe in Lösung, Suspensionen oder Emulsionen gebracht. Als Lösungsmittel für die neuen aktiven Verbindungen und die entsprechenden physiologisch verträglichen Salze kommen z.B. in Frage: Wasser, physiologische Kochsalzlösungen oder Alkohole, z.B. Ethanol, Propandiol oder Glycerin, daneben auch Zuckerlösungen wie Glukose- oder Mannitlösungen, oder auch eine Mischung aus den verschiedenen genannten Lösungsmitteln.

Ebenso können die Wirkstoffe durch Implantate z.B. aus Polylactid, Polyglycolid, Copolymerisate aus Milch- und Glykolsäure oder Poly-3-hydroxybuttersäure bzw. intranasale Zubereitungen appliziert werden. Bei der intranasalen Applikation muß wegen der schlechteren Resorption die Dosis verzehnfacht werden. Der Zusatz eines Chelatbildners, wie z.B. EDTA kann sich vorteilhaft auf die Resorption auswirken. Ferner können die Peptide in Form ihrer physiologisch verträglichen Salze oder Metallkomplexe verabreicht werden.

Beispiel 1:

H-Arg-Ile-Asp(OtBu)-Arg-Ile-OH

a) Z-Arg($Z_2$)-Ile-OBzl

Zu einer Lösung von 5,76 g Z-Arg($Z_2$)-OH, 3,93 g H-Ile-OBzl-tosylat und 1,35 g HOBt in 50 ml Dimethylformamid gibt man bei 0 °C 1,3 ml N-Ethylmorpholin und 2,2 g DCC. Man rührt eine Stunde bei 0 °C und eine Stunde bei Raumtemperatur. Man läßt über Nacht bei Raumtemperatur stehen. Der Niederschlag wird abgesaugt und verworfen. Das Filtrat wird im Vakuum eingeengt und der Rückstand zwischen Essigester und Wasser verteilt. Die Essigesterphase wird nacheinander mit gesättigter wäßriger $NaHCO_3$-Lösung, mit einem $KHSO_4$/$K_2SO_4$-Puffer und Wasser gewaschen. Man trocknet über $Na_2SO_4$ und engt ein. Der Rückstand wird mit Ether verrieben, abgesaugt und getrocknet.

Ausbeute: 7,45 g, Schmp. 142-144 °C, $[\alpha]_D^{22}$ = -5,5 ° (c = 1, in 90-proz. Essigsäure).

b) H-Arg-Ile-OH-acetat

Zu einer Suspension von 7,3 g Z-Arg($Z_2$)-Ile-OBzl in 220 ml 90-proz. Essigsäure gibt man Pd/Kohle-Katalysator und leitet Wasserstoff durch die Lösung. Nach beendigter Reaktion saugt man den Katalysator ab und engt das Filtrat ein. Der Rückstand wird mit Ether verrieben und abgesaugt.

Ausbeute: 3,86 g, Schmp. 197-200 °C, $[\alpha]_D^{22}$ = +19,9 ° (c = 1, 90-proz. Essigsäure).

c) H-Asp(OtBu)-Arg-Ile-OH-acetat

Zu einer Suspension von 3,45 g H-Arg-Ile-OH-acetat in 50 ml Dimethylformamid gibt man 1,35 g HOBt

und 4,2 g Z-Asp (OtBu)-ONSu. Man läßt einen Tag bei Raumtemperatur rühren und engt ein. Der Rückstand wird zwischen 100 ml halbgesättigter wäßriger $NaHCO_3$-Lösung und 100 ml Essigester im Gegenstrom verteilt (5 Stufen). Die Fraktionen, in denen reines Z-Asp(OtBu)-Arg-Ile-OH enthalten ist, werden eingeengt und in 70 ml 90-proz. Essigsäure gelöst und wie im Beispiel 1b katalytisch hydriert.

Ausbeute: 2,22 g, amorph, $[\alpha]_D^{22}$ = +0,8° (c=1, in 90-proz. Essigsäure).

d) Z-Ile-Asp(OtBu)-Arg-Ile-OH

Zu einer Suspension von 8,55 g H-Asp(OtBu)-Arg-Ile-OH-acetat in 80 ml Dimethylformamid gibt man bei Raumtemperatur 2,22 g HOBt und 6,6 g Z-Ile-ONSu. Man rührt 5 Stunden bei Raumtemperatur und engt im Vakuum ein. Der Rückstand wird zwischen halbgesättigter wäßriger $NaHCO_3$-Lösung und Essigester im Gegenstrom verteilt (5 Stufen). Die Fraktionen, in denen reines Z-Ile-Asp(OtBu)-Arg-Ile-OH enthalten ist, werden eingeengt und mit Ether verrieben.

Ausbeute: 10,7 g, Schmp. 177-179° C, $[\alpha]_D^{22}$ = -30,2° (c=1, in 90-proz. Essigsäure).

e) H-Ile-Asp(OtBu)-Arg-Ile-OH-acetat

10,7 g Z-Ile-Asp(OtBu)-Arg-Ile-OH werden analog Beispiel 1b in 150 ml 90-proz. Essigsäure katalytisch hydriert.

Ausbeute: 10,4 g, Schmp. 107-117° C, $[\alpha]_D^{22}$ = -3,5° (c=1, in 90-proz. Essigsäure).

f) Z-Arg($Z_2$)-Ile-Asp(OtBu)-Arg-Ile-OH

Zu einer Lösung von 11,4 g H-Ile-Asp(OtBu)-Arg-Ile-OH-acetat und 2,45 g HOBt in 180 ml Dimethylformamid gibt man 13,6 g Z-Arg($Z_2$)-OTcp. Man läßt 24 Stunden bei Raumtemperatur reagieren und engt ein. Der Rückstand wird mit Essigester verrieben, abgesaugt und getrocknet. Anschließend wird die Substanz mit gesättigter wäßriger $NaHCO_3$-Lösung verrieben, abgesaugt, mit Wasser gewaschen und über $P_2O_5$ getrocknet.

Ausbeute: 5,75 g, Schmp. 184-186° C, $[\alpha]_D^{22}$ = -22,5° (c=1, in 90-proz. Essigsäure).

g) H-Arg-Ile-Asp(OtBu)-Arg-Ile-OH-diacetat

6,34 g Z-Arg($Z_2$)-Ile-Asp(OtBu)-Arg-Ile-OH werden analog Beispiel 1b in 120 ml 90-proz. Essigsäure katalytisch hydriert.

Ausbeute: 5,66 g

Zur Reinigung wird die Substanz zwischen 100 ml n-Butanol und 100 ml 10-proz. Essigsäure im Gegenstrom verteilt (7 Stufen).

Ausbeute: 4,45 g, amorph, $[\alpha]_D^{22}$ = -32,4° (c=1, in Wasser).

Beispiel 2:

H-Arg-Ile-Asp(OtBu)-Arg-Ile-ol

a) L-Isoleucinol•HOObt (= H-Ile-ol•HOObt)

Zu 250 ml auf -5° C gekühltes Tetrahydrofuran gibt man unter Rühren 11,4 g $LiAlH_4$. Zu dieser Suspension gibt man unter Rühren innerhalb von 45 Minuten 28,86 g (0,22 Mol) L-Isoleucin. Anschließend kocht man 4 Stunden unter Rückfluß. Danach kühlt man auf 0° C ab, gibt 250 ml Diethylether zu und läßt unter Rühren langsam 50 ml Wasser zutropfen. Der Niederschlag wird abgesaugt und der Rückstand zweimal mit je 150 ml Methanol ausgekocht und heiß abgesaugt. Die vereinigten Filtrate werden eingeengt. Der Rückstand wird in 100 ml abs. Ethanol aufgenommen und mit 200 ml Ether versetzt. Man läßt über Nacht bei 4° C stehen, saugt den Niederschlag ab und engt das Filtrat ein. Das resultierende Öl wird in 150 ml Methanol gelöst. Dazu gibt man 36 g (0,22 Mol) HOObt und 200 ml Diethylether und läßt das Salz bei 4° C auskristallisieren. Der Niederschlag wird abgesaugt und mit Ether gewaschen.

Ausbeute: 44 g, Schmp. 178-179° C, $[\alpha]_D^{23}$ = +8,8° (c=1, in Methanol).

b) Z-Arg($Z_2$)-Leu-ol

Zu einer Lösung von 17,3 g Z-Arg($Z_2$)-OH und 8,4 g H-Ile-ol. HOObt in 200 ml Dimethylformamid gibt man bei 0°C 6,6 g DCC. Man rührt zwei Stunden bei 0°C und läßt etwa zehn Stunden bei Raumtemperatur stehen. Der Niederschlag wird abgesaugt und verworfen. Das Filtrat wird eingeengt und der Rückstand mit gesättigter wäßriger $NaHCO_3$-Lösung verrieben und abgesaugt, gut mit Wasser gewaschen und getrocknet. Zur Reinigung wird mit Methanol bei Raumtemperatur verrührt, gekühlt, abgesaugt und getrocknet.

Ausbeute: 17,4 g, Schmp. 180-182°C, $[\alpha]_D^{22}$ = -4,2° (c = 1, in Dimethylformamid).

c) H-Arg-Leu-ol-di-tosylat

Zu einer Suspension von 13,5 g Z-Arg($Z_2$)-Leu-ol in 350 ml Methanol gibt man Pd/Kohle-Katalysator und leitet bei pH 4,5 unter Zugabe von 1M p-Toluolsulfonsäure (Autotitrator) Wasserstoff durch. Während der Hydrierung geht die Substanz in Lösung. Nach beendigter Reaktion (p-Toluolsulfonsäure-Lösung wird nicht mehr aufgenommen) saugt man den Katalysator ab und engt ein. Der Rückstand wird mit Wasser verrührt. Unlösliches wird abgesaugt und verworfen. Das Filtrat wird eingeengt und der Rückstand mit Ether verrieben. Der Niederschlag wird abgesaugt und getrocknet.

Ausbeute: 9,95 g, amorphe Substanz, $[\alpha]_D^{22}$ = +1,8° (C = 1, in Methanol).

d) Z-Asp(OtBu)-Arg-Leu-ol-tosylat

Zu einer Lösung von 8,6 g H -Arg-Leu-ol-di-tosylat in 100 ml Dimethylformamid gibt man 1,99 ml N-Ethylmorpholin und 5,85 g Z-Asp(OtBu)-ONSu. Man läßt über Nacht bei Raumtemperatur stehen. Die Lösung wird im Vakuum eingeengt und der Rückstand in n-Pentanol gelöst. Die Lösung wird zweimal mit Wasser ausgeschüttelt. Die organische Phase wird eingeengt und der Rückstand mit Ether verrieben. Der Niederschlag wird abgesaugt.

Ausbeute: 9,05 g, amorphe Substanz, $[\alpha]_D^{22}$ = -26,2° (c = 1, in Methanol).

e) H-Asp(OtBu)-Arg-Leu-ol-di-tosylat

7,5 g Z-Asp(OtBu)-Arg-Leu-ol-tosylat werden in 150 ml Methanol gelöst und analog Beispiel 2c katalytisch hydriert.

Ausbeute: 7,47 g, amorphe Substanz, $[\alpha]_D^{22}$ = -7,4° (c = 1, in Methanol).

f) Z-Arg-Ile-Asp(OtBu)-Arg-Leu-ol-di-tosylat

Zu einer Lösung von 843 mg Z-Arg-Ile-OH, 1,58 g H-Asp (OtBu)-Arg-Leu-ol-di-tosylat und 326 mg HOObt in 20 ml Dimethylformamid gibt man bei 0°C 440 mg DCC. Man rührt 1 Stunde bei 0°C und 2 Stunden bei Raumtemperatur. Über Nacht stellt man bei 4°C und saugt anderntags den Niederschlag ab. Der Niederschlag wird verworfen und das Filtrat im Vakuum eingeengt. Der Rückstand wird zwischen 80 ml Essigsäure und 80 ml Wasser im Gegenstrom verteilt (5 Stufen). Die die Titelverbindung enthaltenden Fraktionen werden eingeengt und zum Schluß gefriergetrocknet.

Ausbeute: 1,89 g, amorphe Substanz, $[\alpha]_D^{20}$ = -29,5° (c = 1, in Methanol).

g) H-Arg-Ile-Asp(OtBu)-Arg-Leu-ol-ditosylat werden in 30 ml 90-proz. Essigsäure gelöst und analog Beispiel 1b katalytisch hydriert. Zur Überführung in das Acetat wird der Rückstand in wäßriger Lösung mit einem schwach basischen Ionenaustauscher (in Acetat-Form) verrührt. Der Austauscher wird abgesaugt und das Filtrat gefriergetrocknet.

Ausbeute: 1,04 g, $[\alpha]_D^{23}$ = -34,7° (c = 1, in Wasser).

Beispiel 3:

H-Arg-Ile-Asp(OtBu)-Arg-Ile-ol

Die Verbindungen wurden analog Beispiel 2 hergestellt.

a) H-Ile-ol • HOObt

Schmp. 178-179°C, $[\alpha]_D^{23}$ = 8,8° (c = 1, in Methanol).

b) Z-Arg($Z_2$)-Ile-ol

Schmp. 180-182° C, $[\alpha]_D^{22}$ = -4,2° (c = 1, in Dimethylformamid).

c) H-Arg-Ile-ol-di-tosylat

Amorphe Substanz, $[\alpha]_D^{22}$ = +7,9° (c = 1, in Methanol).

d) Z-Asp(OtBu)-Arg-Ile-ol-tosylat

Amorphe Substanz, $[\alpha]_D^{23}$ = -22,2° (c = 1, in Methanol).

e) H-Asp(OtBu)-Arg-Ile-ol-di-tosylat

Amorphe Substanz, $[\alpha]_D^{22}$ = -4,2° (c = 1, in Methanol).

f) Z-Arg-Ile-Asp(OtBu)-Arg-Ile-ol-di-tosylat

Amorphe Substanz, $[\alpha]_D^{20}$ = -26,1° (c = 1, in Methanol).

g) H-Arg-Ile-Asp(OtBu)-Arg-Ile-ol-tri-acetat

Amorphe Substanz, $[\alpha]_D^{23}$ = -31,7° (c = 1, in Wasser).

Beispiel 4:

H-Arg-Ile-Asp(OtBu)-Arg-isobutylamid

Die Verbindungen wurden analog Beispiel 2 hergestellt.

a) Z-Arg($Z_2$)-isobutylamid

Schmp. 148-149° C, $[\alpha]_D^{22}$ = +7,7° (c = 1, in Dimethylformamid).

b) H-Arg-isobutylamid-di-tosylat

Amorphe Substanz, $[\alpha]_D^{22}$ = +13,3° (c = 1, in Methanol).

c) Z-Asp(OtBu)-Arg-isobutylamid-tosylat

Amorphe Substanz, $[\alpha]_D^{23}$ = -21,0° (c = 1, in Methanol).

d) H-Asp(OtBu)-Arg-isobutylamid-di-tosylat

Amorphe Substanz, $[\alpha]_D^{22}$ = -1,6° (c = 1, in Methanol).

e) Z-Arg-Ile-Asp(OtBu)-Arg-isobutylamid-di-tosylat

Amorphe Substanz, $[\alpha]_D^{20}$ = -22,9° (c = 1, in Methanol).

f) H-Arg-Ile-Asp(OtBu)-Arg-isobutylamid-tri-acetat

Amorphe Substanz, $[\alpha]_D^{23}$ = -28,5° (c = 1, in Wasser).

Beispiel 5:

H-Arg-Ile-Asp(OtBu)-Arg-Ile-$NH_2$

Die Verbindungen wurden analog Beispiel 2 hergestellt:

a) Z-Arg($Z_2$)-Ile-$NH_2$

Schmp. 178-188 ° C, $[\alpha]_D^{22}$ = +3,5 ° (c = 1, in Dimethylformamid).

b) H-Arg-Ile-$NH_2$-di-tosylat

Amorphe Substanz, $[\alpha]_D^{22}$ = +9,8 ° (c = 1, in Methanol).

c) Z-Asp(OtBu)-Arg-Ile-$NH_2$-tosylat

Amorphe Substanz, $[\alpha]_D^{23}$ = -18,0 ° (c = 1, in Methanol).

d) H-Asp(OtBu)-Arg-Ile-$NH_2$-di-tosylat

Amorphe Substanz, $[\alpha]_D^{22}$ = -3,3 ° (c = 1, in Methanol).

e) Z-Arg-Ile-Asp(OtBu)-Arg-Ile-$NH_2$-di-tosylat

Amorphe substanz, $[\alpha]_D^{20}$ = -26,0 ° (c = 1, in Methanol).

f) H-Arg-Ile-Asp(OtBu)-Arg-Ile-$NH_2$-tri-acetat

Amorphe Substanz, $[\alpha]_D^{23}$ = -34,3 ° (c = 1, in Wasser).

Beispiel 6:

H-Arg-Ile-Asp(OtBu)-Arg-Val-$NH_2$

Die Substanzen wurden analog Beispiel 2 hergestellt.

a) Z-Arg($Z_2$)-Val-$NH_2$

Schmp. 174-178 ° C, $[\alpha]_D^{22}$ = +4,6 ° (c = 1, in Dimethylformamid).

b) H-Arg-Val-$NH_2$-di-tosylat

Amorphe Substanz, $[\alpha]_D^{22}$ = +11,8 ° (c = 1, in Methanol).

c) Z-Asp(OtBu)-Arg-Val-$NH_2$-tosylat

Amorphe Substanz, $[\alpha]_D^{23}$ = -16,5 ° (c = 1, in Methanol).

d) H-Asp(OtBu)-Arg-Val-$NH_2$-di-tosylat

Amorphe Substanz, $[\alpha]_D^{22}$ = 2,7 ° (c = 1, in Methanol).

e) Z-Arg-Ile-Asp(OtBu)-Arg-Val-$NH_2$-di-tosylat

Amorphe Substanz, $[\alpha]_D^{20}$ = -24,3 ° (c = 1, in Methanol).

f) H-Arg-Ile-Asp(OtBu)-Arg-Val-$NH_2$-tri-acetat

Amorphe Substanz, $[\alpha]_D^{23}$ = -36,2 ° (c = 1, in Wasser).

Beispiel 7:

H-Arg-Ile-Asp(OtBu)-Arg-Trp-$NH_2$

Die Substanzen wurden analog Beispiel 2 hergestellt.

a) Z-Arg($Z_2$)-Trp-$NH_2$

Schmp. 172-174 °C, $[\alpha]_D^{22}$ = -1,4 ° (c = 1, in Dimethylformamid).

b) H-Arg-Trp-$NH_2$-di-tosylat

Amorphe Substanz, $[\alpha]_D^{22}$ = +13,5 ° (c = 1, in Methanol).

c) Z-Asp(OtBu)-Arg-Trp-$NH_2$-di-tosylat

Amorphe Substanz, $[\alpha]_D^{23}$ = -18,2 ° (c = 1, in Methanol).

d) H-Asp(OtBu)-Arg-Trp-$NH_2$-di-tosylat

Amorphe Substanz, $[\alpha]_D^{22}$ = -3,2 ° (c = 1, in Methanol).

e) Z-Arg-Ile-Asp(OtBu)-Arg-Trp-$NH_2$-di-tosylat

Amorphe Substanz, $[\alpha]_D^{20}$ = -25,0 ° (c = 1, in Methanol).

f) H-Arg-Ile-Asp(OtBu)-Arg-Trp-$NH_2$-tri-acetat

Amorphe Substanz, $[\alpha]_D^{23}$ = -31,9 ° (c = 1, in Wasser).

Beispiel 8:

H-Arg-Pro-Val-Lys-Val-OH

a) H-Lys(Boc)-Val-OtBu • HCl

Zu einer Lösung von 4,5 g Z-Lys(Boc)-Oh, 2,1 g H-Val-OtBu. HCl und 1,35 g HOBt in 20 ml Dimethylformamid gibt man bei 0 °C unter Rühren 1,3 ml N-Ethylmorpholin und 2,2 g DCC. Man läßt eine Stunde bei 0 °C rühren und anschließend über Nacht bei Raumtemperatur stehen. Der Niederschlag wird abgesaugt und verworfen. Das Filtrat wird im Vakuum eingeengt. Der Rückstand wird zwischen Essigester und Wasser verteilt. Die Essigesterphase wird nacheinander mit $KHSO_4$/$K_2SO_4$-Puffer, gesättigter wäßriger $NaHCO_3$-Lösung und Wasser gewaschen, über $Na_2SO_4$ getrocknet und eingeengt. Reinigung: Chromatographie an Kieselgel in $CH_2Cl_2$/Aceton/Petrolether wie 9:0,5:2.

Ausbeute: 3,7 g ölige Substanz.

Obige Substanz wird in Methanol bei pH 4,5 (Autotitrator: Zugabe von 1N methanolischer HCl) katalytisch (Pd/$BaSO_4$) hydriert. Nach beendigter Hydrierung wird der Katalysator abgesaugt und das Filtrat eingeengt. Der Rückstand wird zwischen n-Pentanol und wäßriger NaCl-Lösung im Gegenstrom verteilt (5 Stufen). Die 1. Pentanolfraktion wird eingeengt und der Rückstand mit Ether verrieben.

Ausbeute: 1,25 g, Schmp. 141-143 °C, $[\alpha]_D^{21}$ = 1,7 ° (c = 1, in Methanol).

b) Z-Pro-Val-OtBu

Zu einer Lösung von 2,49 g Z-Pro-OH, 2,1 g H-Val-OtBu.HCl und 1,35 g HOBt in 20 ml Dimethylformamid gibt man bei 0 °C 1,3 ml N-Ethylmorpholin und 2,2 g DCC. Es wird nun verfahren wie in Beispiel 8a. Der Rückstand wird mit Petrolether verrieben, abgesaugt und getrocknet.

Ausbeute: 2,83 g, Schmp. 110-112 °C, $[\alpha]_D^{21}$ = -66,6 ° (c = 1, in Methanol).

c) Z-Pro-Val-OH

2,63 g Z-Pro-Val-OtBu werden in 20 ml 90-proz. Trifluoressigsäure gelöst. Man läßt eine Stunde bei Raumtemperatur stehen und engt ein. Der Rückstand wird mit Wasser verrieben. Der Niederschlag wird abgesaugt und über $P_2O_5$ getrocknet. Zur weiteren Reinigung chromatographiert man über Kieselgel in

$CH_2Cl_2/CH_3OH$/Petrolether/Essigsäure/Wasser wie 18:1:2: 0,1:0,1.
Ausbeute: 1,2 g, Schmp. 131-133° C, $[\alpha]_D^{22}$ = -56,9° (c = 1, in Methanol).

d) Z-Pro-Val-Lys(Boc)-Val-OtBu

Zu einer Lösung von 1,05 g Z-Pro-Val-OH, 1,3 g H-Lys(Boc)-Val-OtBu.HCl und 0,5 g HOObt in 5 ml Dimethylformamid gibt man bei 0° C 0,38 ml N-Ethylmorpholin und 0,66 g DCC. Man rührt eine Stunde bei 0° C und läßt über Nacht bei Raumtemperatur stehen. Man arbeitet wie in Beispiel 8a auf. Der Rückstand wird mit Ether verrieben. Der Niederschlag wird abgesaugt und getrocknet.
Ausbeute: 1,45 g, Schmp. 139-141° C, $[\alpha]_D^{22}$ = +75,1° (c = 1, in Methanol).

e) H-Pro-Val-Lys(Boc)-Val-OtBu•HCl

1,32 g Z-Pro-Val-Lys(Boc)-Val-OtBu werden analog Beispiel 8a katalytisch hydriert.
Ausbeute: 1 g, Schmp. 150-157° C, $[\alpha]_D^{23}$ = -52,2° (c = 1, in Methanol).

f) Z-Arg($Z_2$)-Pro-Val-Lys(Boc)-Val-OtBu

Zu einer Lösung von 0,89 g H-Pro-Val-Lys(Boc)-Val-OtBu•HCl, 0,81 g Z-Arg($Z_2$)-OH und 0,23 g HOObt in 10 ml Dimethylformamid gibt man bei 0° C 0,18 ml N-Ethylmorpholin und 0,31 g DCC und verfährt weiter wie in Beispiel 8a. Der Rückstand kristallisiert beim Verreiben mit Ether.
Ausbeute: 1,02 g, Schmp. 86-92° C, $[\alpha]_D^{23}$ = -56,3° (c = 1, in Methanol).

g) H-Arg-Pro-Val-Lys-Val-OH-di-acetat

0,9 g Z-Arg($Z_2$)-Pro-Val-Lys(Boc)-Val-OtBu werden in 90-proz. Essigsäure suspendiert und analog Beispiel 1b katalytisch hydriert. Der etherunlösliche Rückstand (0,55 g) wird in 5 ml 90-proz. Trifluoressigsäure gelöst. Man läßt eine Stunde bei Raumtemperatur stehen und engt ein. Der Rückstand wird in Wasser aufgenommen und mit schwach basischem Ionenaustauscher (in Acetat-Form) verrührt. Der Ionenaustauscher wird abgesaugt und das Filtrat gefriergetrocknet. Reinigung: Chromatographie an einem vernetzten Dextrangel.
Ausbeute: 306,3 mg, $[\alpha]_D^{24}$ = +89,5° (c = 1, in Wasser).

Beispiel 9:

H-D-Arg-Ile-Leu-Arg-Ile-OH

a) Z-D-Arg-Ile-OBzl

Zu einer Lösung von 9,25 g Z-D-Arg-OH, 11,8 g H-Ile-OBzl-tosylat und 4,05 g HOBt in 150 ml Dimethylformamid gibt man bei 0° C 6,6 g DCC. Man rührt eine Stunde bei 0° C und läßt über Nacht bei Raumtemperatur stehen. Der Niederschlag wird abgesaugt und verworfen. Das Filtrat wird eingeengt und der Rückstand zwischen Essigester und gesättigter wäßriger $NaHCO_3$-Lösung verteilt. Die Essigesterphase wird mit Wasser gewaschen, über $Na_2SO_4$ getrocknet und eingeengt. Der Rückstand wird mit Ether verrieben. Man dekantiert ab und verreibt mit Petrolether. Der Niederschlag wird abgesaugt und getrocknet.
Ausbeute: 19,5 g; $[\alpha]_D^{25}$ = -2,7° (c = 1, in Methanol).

b) Z-D-Arg-Ile-OH

Eine Lösung von 19 g Z-D-Arg-Ile-OBzl in einer Mischung aus 70 ml Dioxan, 30 ml Wasser und 12 ml Methanol versetzt man mit 41,8 ml wäßriger 1N NaOH und läßt über Nacht bei Raumtemperatur stehen. Anderntags neutralisiert man mit ca. 3,3 ml 4N HCl. Die trübe Lösung wird über eine Klärschicht gesaugt und das Filtrat eingeengt. Den Rückstand verteilt man zwischen 120 ml n-Pentanol und 120 ml Wasser. Die wäßrige Phase wird noch 4 mal mit n-Pentanol ausgeschüttelt. Die n-Pentanolphasen werden zusammen eingeengt. Der Rückstand wird mit Essigester verrieben und der Niederschlag abgesaugt.
Ausbeute: 8,4 g, $[\alpha]_D^{22}$ = +12,3° (c = 1, in 90-proz. Essigsäure).

c) Fmoc-Leu-OObt

Zu einer Lösung von 3,53 g Fmoc-Leu-OH und 1,63 g HOObt in 40 ml $CH_2Cl_2$ gibt man 2,06 g DCC. Man rührt 1 Stunde bei 0 ° C und läßt über Nacht bei Raumtemperatur stehen. Der Niederschlag wird abgesaugt und verworfen. Das Filtrat wird eingeengt und der Rückstand zweimal mit Petrolether verrieben und am Hochvakuum getrocknet.

Ausbeute: 5,2 g amorphe Substanz.

d) Fmoc-Leu-Arg-OH

Zu einer Mischung aus 1,74g Arginin, 1,63 g HOObt und 1,79 g Pyridiniumperchlorat in 50 ml Dimethylformamid gibt man bei Raumtemperatur 5 g Fmoc-Leu-OObt. Man rührt 3 Stunden bei Raumtemperatur und läßt über Nacht bei Raumtemperatur stehen. Die klare Lösung wird eingeengt und der Rückstand mit Wasser versetzt. Mit wäßriger $NaHCO_3$-Lösung wird ein pH von 7 eingestellt. Der Niederschlag wird abgesaugt, mit Wasser gewaschen und getrocknet.

Ausbeute: 4,7 g, Schmp. 145-155 ° C (unter Zersetzung), $[\alpha]_D^{23}$ = -18,5 ° (c = 1, in Methanol).

e) Fmoc-Leu-Arg-Ile-OBzl

Zu einer Lösung von 2,04 g Fmoc-Leu-Arg-OH, 1,57 g H-Ile-OBzl-tosylat und 0,65 g HOObt in 20 ml Dimethylformamid gibt man bei 0 ° C 880 mg DCC. Man rührt eine Stunde bei 0 ° C und läßt über Nacht bei Raumtemperatur stehen. Der Niederschlag wird abgesaugt und verworfen. Das Filtrat wird eingeengt und der Rückstand wird zwischen Essigester und gesättigter wäßriger $NaHCO_3$-Lösung verteilt. Die Essigesterphase wird noch zweimal mit gesättigter wäßriger $NaHCO_3$-Lösung und 3 mal mit Wasser ausgeschüttelt, über $Na_2SO_4$ getrocknet und eingeengt. Der Rückstand wird mit Ether verrieben und abgesaugt.

Ausbeute: 2,59 g, $[\alpha]_D^{24}$ = -36,4 ° (c = 1, in Methanol).

f) Z-D-Arg-Ile-Leu-Arg-Ile-OBzl-di-acetat

Zu einer Lösung von 2,18 g Fmoc-Leu-Arg-Ile-OBzl in 15 ml Dimethylformamid gibt man 3,25 ml Diethylamin. Man läßt 15 Minuten bei Raumtemperatur rühren und engt ein. Der Rückstand wird in 125 ml n-Pentanol gelöst und mit einer halbkonzentrierten wäßrigen $NaHCO_3$-Lösung ausgeschüttelt. Die n-Pentanolphase wird eingeengt und der Rückstand mit Ether verrieben und abgesaugt.

Ausbeute: 1,4 g H-Leu-Arg-Ile-OBzl.

Zu einer Lösung von 421,5 mg Z-D-Arg-Ile-OH, 490,6 mg H-Leu-Arg-Ile-OBzl, 179,5 mg Pyridiniumperchlorat und 163 mg HOObt in 10 ml Dimethylformamid gibt man bei 0 ° C 220 mg DCC. Man rührt eine Stunde bei 0 ° C und läßt über Nacht bei Raumtemperatur stehen. Der Niederschlag wird abgesaugt und verworfen. Das Filtrat wird eingeengt und der Rückstand in 50 ml n-Pentanol gelöst. Die Lösung wird 2 mal mit je 50 ml halbkonzentrierter wäßriger $NaHCO_3$-Lösung ausgeschüttelt und eingeengt. Der Rückstand wird mit Essigsäure verrieben und abgesaugt.

Ausbeute: 865 mg.

Zur Reinigung wird die Substanz zwischen 45 ml 10-proz. wäßriger Essigsäure und 45 ml Essigester im Gegenstrom verteilt. Die wäßrigen Phasen 1-4 werden eingeengt und der Rückstand mit Ether verrieben und abgesaugt.

Ausbeute: 620 mg, amorphe Substanz, $[\alpha]_D^{23}$ = -31,3 ° (c = 1, in Methanol).

g) H-D-Arg-Ile-Leu-Arg-Ile-OH-di-acetat

510 mg Z-D-Arg-Ile-Leu-Arg-Ile-OBzl werden analog Beispiel 1b in 15 ml 90-proz. Essigsäure katalytisch hydriert.

Ausbeute: 445 mg, $[\alpha]_D^{21}$ = -66,2 ° (c = 1, in Wasser).

Beispiel 10:

H-Arg-Ile-Leu-D-Arg-Ile-OH

a) Fmoc-Leu-D-Arg-OH

Die Substanz wird analog Beispiel 9d hergestellt.
Schmp. 145-155 ° C (unter Zersetzung), $[\alpha]_D^{23}$ = -31,3 ° (c = 1, in Methanol).

b) Fmoc-Leu-D-Arg-Ile-OBzl

Die Substanz wird analog Beispiel 9e hergestellt.
$[\alpha]_D^{24}$ = -9,8° (c=1, in Methanol).

c) Z-Arg-Ile-Leu-D-Arg-Ile-OBzl-di-acetat

Die Substanz wird analog Beispiel 9f hergestellt.
Amorphe Substanz, $[\alpha]_D^{23}$ = -18,3° (c=1, in Methanol).

d) H-Arg-Ile-Leu-D-Arg-Ile-OH-di-acetat

Die Substanz wird analog Beispiel 9g hergestellt.
Amorphe Substanz, $[\alpha]_D^{24}$ = -14,0° (c=1, in Wasser).

Beispiel 11:

H-Arg-Ile-Leu-Arg-D-Val-OH

a) Fmoc-Leu-Arg-D-Val-OBzl

Die Substanz wird analog Beispiel 9e hergestellt.
Amorphe Substanz, $[\alpha]_D^{24}$ = -18,3° (c=1, in Methanol).

b) Z-Arg-Ile-Leu-Arg-D-Val-OBzl-di-acetat

Die Substanz wird analog Beispiel 9f hergestellt.
Amorphe Substanz, $[\alpha]_D^{23}$ = -24,2° (c=1, in Methanol).

c) H-Arg-Ile-Leu-Arg-D-Val-OH-di-acetat

Die Substanz wird analog Beispiel 9g hergestellt.
Amorphe Substanz, $[\alpha]_D^{20}$ = -37,1° (c=1, in Wasser).

Beispiel 12:

H-Arg-Ile-D-Leu-Arg-D-Val-OH

a) Fmoc-D-Leu-Arg-OH

Die Substanz wird analog Beispiel 9d hergestellt.
Schmp. 140-150°C (unter Zersetzung), $[\alpha]_D^{23}$ = +30,8° (c=1, in Methanol).

b) Fmoc-D-Leu-Arg-D-Val-OBzl

Die Substanz wird analog Beispiel 9e hergestellt.
Amorphe Substanz, $[\alpha]_D^{24}$ = +11,2° (c=1, in Methanol).

c) Z-Arg-Ile-D-Leu-Arg-D-Val-OBzl-di-acetat

Die Substanz wird analog Beispiel 9f hergestellt.
Amorphe Substanz, $[\alpha]_D^{23}$ = +5,2° (c=1, in Methanol).

d) H-Arg-Ile-D-Leu-Arg-D-Val-OH-di-acetat

Die Substanz wird analog Beispiel 9g hergestellt.
Amorphe Substanz, $[\alpha]_D^{22}$ = +10,6° (c=1, in Wasser).

Beispiel 13:

H-D-Arg-Ile-D-Leu-Arg-Ile-OH

a) Fmoc-D-Leu-Arg-Ile-OBzl

Die Substanz wird analog Beispiel 9e hergestellt.
Amorphe Substanz, $[\alpha]_D^{24}$ = -5,7° (c=1, in Methanol).

b) Z-D-Arg-Ile-D-Leu-Arg-Ile-OBzl-di-acetat

Die Substanz wird analog Beispiel 9f hergestellt.
Amorphe Substanz, $[\alpha]_D^{24}$ = -2,4° (c=1, in Methanol).

c) H-D-Arg-Ile-D-Leu-Arg-Ile-OH-di-acetat

Die Substanz wird analog Beispiel 9g hergestellt.
Amorphe Substanz, $[\alpha]_D^{23}$ = -20,3° (c=1, in Wasser).

Beispiel 14:

H-D-Arg-D-allo-Ile-D-Leu-Arg-Ile-OH-di-acetat

Bei der Reinigung von H-D-Arg-Ile-D-Leu-Arg-Ile-OH durch Säulenchromatographie wurde eine Fraktion isoliert, die laut Aminosäureanalyse H-D-Arg-D-allo-Ile-D-Leu-Arg-Ile-OH war. Amorphe Substanz, Aminosäureanalyse: D-allo-Ile (0,9), Ile (1,06), Leu (1,03), Arg (1,87).

Beispiel 15:

H-D-Arg-Ile-Leu-D-Arg-Ile-OH

a) Z-D-Arg-Ile-Leu-D-Arg-Ile-OBzl-di-acetat

Die Substanz wird analog Beispiel 9f hergestellt.
Amorphe Substanz, $[\alpha]_D^{24}$ = -6,9° (c=1, in Methanol).

b) H-D-Arg-Ile-Leu-D-Arg-Ile-OH-di-acetat

Die Substanz wird analog Beispiel 9g hergestellt.
Amorphe Substanz, $[\alpha]_D^{23}$ = -30,5° (c=1, in Wasser).

Beispiel 16:

H-Arg-D-Val-Leu-Arg-Ile-OH

a) Z-Arg-D-Val-OBzl

Die Substanz wird analog Beispiel 9a hergestellt.
Amorphe Substanz, $[\alpha]_D^{25}$ = +6,2° (c=1, in Methanol).

b) Z-Arg-D-Val-OH

Die Substanz wird analog Beispiel 9b hergestellt.
$[\alpha]_D^{24}$ = -6,1° (c=1, in 90-proz. Essigsäure).

c) Z-Arg-D-Val-Leu-Arg-Ile-OBzl-di-acetat

Die Substanz wird analog Beispiel 9f hergestellt.

Amorphe Substanz, $[\alpha]_D^{23}$ = -30,7° (c=1, in Methanol).

d) H-Arg-D-Val-Leu-Arg-Ile-OH-di-acetat

Die Substanz wird analog Beispiel 9g hergestellt.
Amorphe Substanz, $[\alpha]_D^{19}$ = -15,1° (c=1, in Wasser).

Beispiel 17:

H-Arg-Ile-Trp-Arg-Ile-OH

a) Z-Arg-Ile-OH

Die Substanz wird analog Beispiel 9a und 9b hergestellt.
$[\alpha]_D^{22}$ = -5,9° (c=1, in Methanol).

b) Z-Arg($Z_2$)-Ile-OMe

Die Substanz wird analog Beispiel 1a hergestellt.
Schmp. 129-130°C.

c) H-Arg-Ile-OMe-di-tosylat

Die Substanz wird durch katalytische Hydrierung von Z-Arg($Z_2$)-Ile-OMe analog Beispiel 2c hergestellt.
Amorphe Substanz, $[\alpha]_D^{22}$ = +8,4° (c=1, in Methanol).

d) Z-Trp-Arg-Ile-OMe-tosylat

Die Substanz wird analog Beispiel 2d hergestellt.
Amorphe Substanz, $[\alpha]_D^{22}$ = -20,6° (c=1, in Methanol).

e) H-Trp-Arg-Ile-OMe-di-tosylat

Die Substanz wird analog Beispiel 2e hergestellt.
Amorphe Substanz, $[\alpha]_D^{22}$ = -4,5° (c=1, in Methanol).

f) Z-Arg-Ile-Trp-Arg-Ile-OMe-di-tosylat

Die Substanz wird analog Beispiel 2f hergestellt.
Amorphe Substanz, $[\alpha]_D^{22}$ = -27,5° (c=1, in Methanol).

g) Z-Arg-Ile-Trp-Arg-Ile-OH-tosylat

Zu einer Lösung von 2,7 g Z-Arg-Ile-Trp-Arg-Ile-OMe in 30 ml Dioxan, 10 ml Wasser und 4 ml Methanol gibt man unter Rühren bis pH 13,5 tropfenweise wäßrige NaOH. Es wurden ingesamt 5,2 ml 1N NaOH verbraucht. Mit 0,82 ml wäßriger 1N p-Toluolsulfonsäure-Lösung stellt man die Lösung auf pH 7,8 und engt im Vakuum ein. Der Rückstand wird in n-Butanol gelöst und 3 mal mit Wasser extrahiert. Die vereinigten Wasser-Phasen werden einmal mit n-Butanol extrahiert. Die vereinigten n-Butanol-Phasen werden eingeengt. Der Rückstand wird in Ethanol gelöst und in eine Mischung aus Essigester und Ether eingetropft.
Ausbeute: 1,59 g, $[\alpha]_D^{22}$ = -25,2° (c=1, in Methanol).

h) H-Arg-Ile-Trp-Arg-Ile-OH-di-tosylat

Die Verbindung wird durch katalytische Hydrierung von Z-Arg-Ile-Trp-Arg-Ile-OH-tosylat analog Beispiel 2c hergestellt.
Amorphe Substanz, $[\alpha]_D^{22}$ = -10,5° (c=1, in Methanol).

Unter den gleichen Bedingungen wie bei Beispiel 17 wurden noch folgende Peptide hergestellt:
Z-Arg-Ile-Leu-Arg-Ile-OMe-di-tosylat

Z-Arg-Ile-Leu-Arg-Ile-OH-tosylat
H-Arg-Ile-Leu-Arg-Ile-OH-di-tosylat
Z-Arg-Ile-Phe-Arg-Ile-OMe-di-tosylat
Z-Arg-Ile-Phe-Arg-Ile-OH-tosylat
H-Arg-Ile-Phe-Arg-Ile-OH-di-tosylat
Z-Arg-Ile-Ser(tBu)-Arg-Ile-OMe-di-tosylat
Z-Arg-Ile-Ser(tBu)-Arg-Ile-OH-tosylat
H-Arg-Ile-Ser(tBu)-Arg-Ile-OH-di-tosylat
Z-Arg-Ile-D-Ser(tBu)-Arg-Ile-OMe-di-tosylat
Z-Arg-Ile-D-Ser(tBu)-Arg-Ile-OH-tosylat
H-Arg-Ile-D-Ser(tBu)-Arg-Ile-OH-di-tosylat.

Die Aminosäureanalyse ist jeweils in Übereinstimmung mit den erwarteten Werten.

Beispiel 18:

cyclo- (Gly-Arg-Ile-Phe-Arg-Ile)

Zu einer Lösung von 210 mg H-Arg-Ile-Phe-Arg-Ile-OH-di-tosylat in 3 ml Dimethylformamid gibt man bei Raumtemperatur 78 mg Boc-Gly-OTcp, 3 mg HOBt und 0,026 ml N-Ethylmorpholin. Man rührt einige Stunden bei Raumtemperatur und läßt über Nacht bei Raumtemperatur stehen. Man engt im Vakuum ein und verteilt den Rückstand zwischen Wasser und Essigester, dem wenig Ethanol zugefügt wurde. Die Essigesterphase wird noch einmal mit Wasser extrahiert. Die vereinigten Wasserphasen werden mit Essigester extrahiert. Die vereinigten Wasserphasen werden eingeengt. Der Rückstand (Boc-Gly-Arg-Ile-Phe-Arg-Ile-OH-tosylat) wird in 2 ml Trifluoressigsäure gelöst. Nach 45 Minuten bei Raumtemperatur wird eingeengt und 2 mal mit Ether nachdestilliert. Es bleiben 200 mg eines Harzes zurück (H-Gly-Arg-Ile-Phe-Arg-Ile-OH-tosylat-trifluoracetat), das in 200 ml Dimethylformamid gelöst wird. Dazu gibt man 0,1 ml einer 50-proz. Lösung von Ethylmethylphosphinsäureanhydrid in $CH_2Cl_2$ und unter Rühren langsam eine Lösung von 0,16 ml N-Ethylmorpholin in 5 ml Dimethylformamid. Man rührt 2,5 Stunden bei Raumtemperatur, gibt wenig Wasser zu und engt im Vakuum ein. Der Rückstand wird in wenig Isopropanol gelöst und unter Rühren in Ether eingetropft.

Ausbeute: 60 mg. Das FAB-Massenspektrum zeigt die erwartete Molmasse von 743.

Beispiel 19:

cyclo-(Gly-Arg-Ile-Leu-Arg-Ile)

Analog Beispiel 18 wird aus H-Arg-Ile-Leu-Arg-Ile-OH-di-tosylat die Verbindung hergestellt. Das FAB-Massenspektrum zeigt die erwartete Molmasse von 708.

Beispiel 20:

H-Arg-Leu-Gln-Arg-Leu-OH

a) H-Arg-Leu-OtBu•2 HCl

Zu einer Lösung von 6,7 g H-Leu-OtBu•HCl und 4,05 g HOBt in 50 ml Dimethylformamid gibt man bei Raumtemperatur 3,9 ml N-Ethylmorpholin und 22,7 g Z-Arg($Z_2$)-OTcp. Nach zwei Stunden Reaktionszeit wird der Ansatz mit 300 ml Eis-Wasser versetzt. Der Niederschlag wird abgesaugt, mit gesättigter wäßriger $NaHCO_3$-Lösung verrieben, erneut abgesaugt und mit Wasser gewaschen und getrocknet.

Ausbeute: 27,4 g Z-Arg($Z_2$)-Leu-OtBu. Die oben gewonnene Substanz wird analog Beispiel 8a katalytisch hydriert.

Ausbeute: 11,6 g, $[\alpha]_D^{23}$ = -4,0° (c = 1, in Methanol).

b) H-Arg-Leu-Gln-Arg-Leu-OH-di-acetat

Zu einer Lösung von 3,27 g Z-Arg($Z_2$)-Leu-Gln-OH, 1,66 g H-Arg-Leu-OtBu•2 HCl, 652 mg HOObt in 50 ml Dimethylformamid gibt man bei 0° C 0,52 ml N-Ethylmorpholin und 880 mg DCC. Man rührt eine Stunde bei 0° C und eine Stunde bei Raumtemperatur und läßt über Nacht bei Raumtemperatur stehen. Der

Niederschlag wird abgesaugt. Aus dem Filtrat wird das Peptid mit halbgesättigter wäßriger $NaHCO_3$-Lösung ausgefällt. Der Niederschlag (Z-Arg($Z_2$)-Leu-Gln-Arg-Leu-OtBu) wird abgesaugt und mit Wasser gewaschen und getrocknet.
Die oben gewonnene Substanz wird in 90-proz. Essigsäure analog Beispiel 1b katalytisch hydriert.

Ausbeute: 3,7 g H-Arg-Leu-Gln-Arg-Leu-OtBu-tri-acetat.

300 mg des H-Arg-Leu-Gln-Arg-Leu-OtBu-tri-acetats werden in 3 ml 90-proz. Trifluoressigsäure gelöst. Man läßt eine Stunde bei Raumtemperatur stehen und engt im Vakuum ein. Der Rückstand wird in Wasser gelöst und mit einem schwach basischen Ionenaustauscher (Acetat-Form) verrührt, bis sich ein pH von etwa 4 eingestellt hat. Der Ionenaustauscher wird abgesaugt und das Filtrat gefriergetrocknet.

Ausbeute: 258,8 mg, $[\alpha]_D^{23}$ = -28,9° (c=1, in Wasser).

Beispiel 21:

H-Arg-Val-Tyr-Arg-Pro-OH

a) Z-Tyr(tBu)-Arg-Pro-OtBu

H-Arg-Pro-OtBu-di-tosylat wird analog Beispiel 20a und Beispiel 2c hergestellt.

Zu einer Lösung von 3,4 g H-Arg-Pro-OtBu-di-tosylat, 1,9 g Z-Tyr(tBu)-OH und 0,69 g HOBt in 15 ml Dimethylformamid gibt man bei 0°C 0,66 ml N-Ethylmorpholin und 1,12 g DCC. Man läßt eine Stunde bei 0°C rühren und stellt anschließend über Nacht bei Raumtemperatur. Der Niederschlag wird abgesaugt und verworfen. Das Filtrat wird eingeengt und der Rückstand zwischen Essigester und gesättigter wäßriger $NaHCO_3$-Lösung verteilt. Die Essigesterphase wird noch einmal mit gesättigter wäßriger $NaHCO_3$-Lösung und einmal mit Wasser ausgeschüttelt, über $Na_2SO_4$ getrokknet und eingeengt. Der Rückstand wird mit Petrolether verrieben und abgesaugt.

Ausbeute: 3,35 g, Schmp. 115-125°C (unter Zersetzung), $[\alpha]_D^{24}$ = -35,8° (c=1, in Methanol).

b) H-Tyr(tBu)-Arg-Pro-OtBu-di-tosylat

Die Substanz wird analog Beispiel 2c hergestellt.
Schmp. 105-110°C (unter Zersetzung), $[\alpha]_D^{24}$ = -20,7° (c=1, in Methanol).

c) Z-Arg($Z_2$)-Val-OtBu

Zu einer Lösung von 5,77 g Z-Arg($Z_2$)-OH, 2,1 g H-Val-OtBu•HCl und 1,63 g HOObt in 20 ml Dimethylformamid gibt man bei 0°C 1,3 ml N-Ethylmorpholin und 2,2 g DCC. Man verfährt anschließend wie in Beispiel 1a.

Ausbeute: 4,55 g, Schmp. 103-105°C, $[\alpha]_D^{24}$ = +9,2° (c=1, in Methanol).

d) Z-Arg($Z_2$)-Val-OH

4,5 g Z-Arg($Z_2$)-Val-OtBu werden in ca. 45 ml 90-proz. Trifluoressigsäure gelöst. Man läßt eine Stunde bei Raumtemperatur stehen und engt ein. Der Rückstand wird mit Ether verrieben und abbesaugt.

Ausbeute: 3,58 g, Schmp. 142-144°C $[\alpha]_D^{22}$ = +1,1° (c=1, in Methanol).

e) Z-Arg($Z_2$)-Val-Tyr(tBu)-Arg-Pro-OtBu

Zu einer Lösung von 2,8 g H-Tyr(tBu)-Arg-Pro-OtBu-di-tosylat, 2,1 g Z-Arg($Z_2$)-Val-OH und 0,5 g HOObt in 10 ml Dimethylformamid gibt man bei 0°C 0,4 ml N-Ethylmorpholin und 0,68 g DCC. Man rührt eine Stunde bei 0°C und läßt über Nacht bei Raumtemperatur stehen. Der Niederschlag wird abgesaugt und verworfen. Das Filtrat wird im Vakuum eingeengt. Der Rückstand wird zwischen Essigester und Wasser verteilt. Dabei fällt eine Substanz aus, die abgesaugt wird. Diese Substanz wird zweimal mit gesättigter wäßriger $NaHCO_3$-Lösung und einmal mit Wasser verrieben und jeweils abgesaugt. Die Substanz wird aus Essigester umkristallisiert.

Ausbeute: 2,6 g, Schmp. 141-147°C, $[\alpha]_D^{23}$ = -41,8° (c=1, in Methanol).

f) H-Arg-Val-Tyr(tBu)-Arg-Pro-OtBu-tri-acetat

2,4 g Z-Arg($Z_2$)-Val-Tyr(tBu)-Arg-Pro-OtBu werden analog Beispiel 1b katalytisch hydriert.
Ausbeute nach Reinigung über ein Dextrangel: 1,172 g, $[\alpha]_D^{23}$ = -39,2° (c=1, in Wasser).

g) H-Arg-Val-Tyr-Arg-Pro-OH-di-acetat

670 mg H-Arg-Val-Tyr(tBu)-Arg-Pro-OtBu-tri-acetat werden in einer Mischung aus 10 ml 90-proz. Trifluoressigsäure und 1 ml Ethylmercaptan gelöst. Man läßt eine Stunde bei Raumtemperatur stehen und engt im Vakuum ein. Der Rückstand wird zwischen Wasser und tert.-Butyl-methyl-ether verteilt. Die wäßrige Phase wird mit einem schwach basischem Ionenaustauscher (Acetat-Form) verrührt, bis sich ein pH von 3,5-4,0 eingestellt hat. Der Ionenaustauscher wird abgesaugt und das Filtrat gefriergetrocknet.
Ausbeute nach Reinigung über ein Dextrangel: 310 mg. Die Aminosäureanalyse zeigte die berechneten Werte.

Beispiel 22:

H-Lys-Ile-Asp(OtBu)-Arg-Ile-$NH_2$-ditosylat

a) Z-Lys(Z)-Ile-OMe

29,8 g Z-Lys(Z)-OH•DCHA und 9,08 g H-Ile-OMe•HCl werden in DMF gelöst und nach 15 min. vom ausgefallenen DCHA•HCl abgesaugt. Das Filtrat wird mit 10,1 g HOBt und bei 0°C mit 10,8 g DCC versetzt. Man läßt 1 Stunde bei 0°C und eine weitere Stunde bei Raumtemperatur reagieren und zieht dann das Lösungsmittel ab. Der Rückstand wird in Essigsäure aufgenommen, vom Harnstoff abfiltriert und das Filtrat nacheinander mit wäßriger $NaHCO_3$- und NaCl-lösung ausgeschüttelt. Nach Trocknung über $MgSO_4$ wird die organische Phase eingeengt und der Rückstand aus Essigester/Hexan umkristallisiert.
Ausbeute: 25,1 g, Schmp. 103-105°C, $[\alpha]_D^{20}$ = -13,8° (c=1, MeOH).

b) Z-Lys(Z)-Ile-OH

23,3 g Z-Lys(Z)-Ile-OMe werden in 150 ml Methanol gelöst und unter Rühren mit 50 ml 2N wäßriger NaOH versetzt. Nach vollständiger Reaktion wird das Lösungsmittel abgezogen, der Rückstand in Wasser gelöst und mit Essigester extrahiert. Die Wasserphase wird unter Kühlung auf pH 2-3 angesäuert und mit Essigester extrahiert. Die organische Phase wird mit Wasser gewaschen, über $MgSO_4$ getrocknet und der nach Abziehen des Lösungsmittels erhaltene Rückstand aus Essigester/Diisopropylether umkristallisiert.
Ausbeute: 17,5 g, Schmp. 110-112°C (Zersetzung),
$[\alpha]_D^{20}$ = + 2,4° (c=1, DMF).

c) Z-Lys(Z)-Ile-Asp(OtBu)-Arg-Ile-$NH_2$

Darstellung erfolgt analog Beispiel 2f aus den Verbindungen der Beispiele 22b und 5d. Amorphe Substanz mit korrektem FAB-Massenspektrum (967, M+H).

d) H-Lys-Ile-Asp(OtBu)-Arg-Ile-$NH_2$-ditosylat

Darstellung erfolgt analog Beispiel 2c aus der Verbindung des Beispiels 22c. Amorphe Substanz mit korrektem FAB-Massenspektrum (699, M+H).
$[\alpha]_D^{20}$ = -23,4° (c=1, MeOH)

Beispiel 23:

H-$\epsilon$-Aminohexanoyl-Ile-Asp(OtBu)-Arg-Ile-$NH_2$-ditosylat

Darstellung erfolgt analog Beispiel 22.

a) Z-$\epsilon$-Aminohexanoyl-Ile-OMe

Öliger Sirup, $[\alpha]_D^{20}$ = -6,9° (c=1, MeOH)

b) Z-ε-Aminohexanoyl-Ile-OH

Schmp. 73-75 ° C (Zersetzung), $[\alpha]_D^{20}$ = +5,1 ° (c = 1, DMF)

c) Z-ε-Aminohexanoyl-Ile-Asp(OtBu)-Arg-Ile-$NH_2$

Amorphe Substanz mit korrektem FAB-Massenspektrum (819, M + H)

d) H-ε-Aminohexanoyl-Ile-Asp(OtBu)-Arg-Ile-$NH_2$-ditosylat

Amorphe Substanz mit korrektem FAB-Massenspektrum (684, M + H) $[\alpha]_D^{20}$ = -20,6 ° (c = 1, MeOH).

Beispiel 24:

H-Arg-Ile-Glu(OtBu)-Arg-Ile-$NH_2$-ditosylat-acetat

Darstellung erfolgt analog Beispiel 2.

a) Z-Glu(OtBu)-Arg-Ile-$NH_2$-tosylat wurde analog Beispiel 2d dargestellt.

Amorphe Substanz mit korrektem NMR-Spektrum.

b) H-Glu(OtBu)-Arg-Ile-$NH_2$-ditosylat

Darstellung erfolgt durch katalytische Hydrierung analog Beispiel 2c. Die so erhaltene amorphe Substanz wurde sofort weiter umgesetzt.

c) Z-Arg-Ile-Glu(OtBu)-Arg-Ile-$NH_2$-tosylat-acetat

Darstellung erfolgt analog Beispiel 2f aus der Verbindung des Beispiels 24b und Z-Arg-Ile-OH. Die Endreinigung wird mittels Säulenchromatographie an Kieselgel mit $CH_2Cl_2$/MeOH/$H_2O$/AcOH (20:7:2:2) durchgeführt.
Amorphe Substanz mit korrektem FAB-Massenspektrum (876, M + H) $[\alpha]_D^{20}$ = -23,9 ° (c = 1, MeOH)

d) H-Arg-Ile-Glu(OtBu)-Arg-Ile-$NH_2$-ditosylat-acetat

Darstellung erfolgt durch katalytische Hydrierung analog Beispiel 2c.
Amorphe Substanz mit korrektem FAB-Massenspektrum (741, M + H) $[\alpha]_D^{20}$ = -15,3 ° (c = 1,05; MeOH)

Beispiel 25:

H-Arg-Ile-Ser(tBu)-Arg-Pro-OtBu

a) Z-Ser(tBu)-Arg-Pro-OtBu-tosylat

Zu einer Lösung von 4,43 g Z-Ser(tBu)-OH (15 mmol), 10,03 g H-Arg-Pro-OtBu-ditosylat und 2,02 g HOBt in 40 ml Dimethylformamid gibt man bei 0 ° C unter Rühren 3,3 g DCC. Der Ansatz wird analog Beispiel 1a aufgearbeitet.
Ausbeute 9,8 g, amorph, $[\alpha]_D^{20}$ = -49,5 ° (c = 1, in Methanol).

b) H-Ser(tBu)-Arg-Pro-OtBu-ditosylat

9,2 g Z-Ser(tBu)-Arg-Pro-OtBu-tosylat werden analog Beispiel 2c katalytisch hydriert.
Ausbeute 8,85 g, amorph, $[\alpha]_D^{20}$ = -40,8 ° (c = 1, in Wasser).

c) Z-Arg-Ile-Ser(tBu)-Arg-Pro-OtBu-diacetat

Zu einer Suspension von 4,21 g Z-Arg-Ile-OH (10 mmol), 8,15 g H-Ser(tBu)-Arg-Pro-OtBu-ditosylat und

1,63 g HOObt in 50 ml Dimethylformamid gibt man bei 0°C unter Rühren 2,2 g DCC. Man läßt 1 Stunde bei 0°C und 2 Stunden bei Raumtemperatur rühren, läßt über Nacht stehen und saugt anderntags den Niederschlag ab. Das Filtrat wird eingeengt und der Rückstand zwischen n-Pentanol und halbkonz. $NaHCO_3$-Lösung verteilt. Die Pentanol-phase wird eingeengt und der Rückstand mit Essigester verrieben.

Ausbeute 11 g.

Oben gewonnene Substanz wird in wäßriger Essigsäure gelöst und über einen schwach basischen Ionenaustauscher in Acetat-Form chromatographiert. Das Peptid-haltige Eluat wird vereinigt und gefriergetrocknet.

Ausbeute 8,75 g.

500 mg oben gewonnener Substanz wird zur Reinigung über Kieselgel chromatographiert. Eluiert wird mit einer Mischung aus 1560 ml n-Butanol, 181 ml Essigsäure und 550 ml Wasser.

Ausbeute 251 mg, $[\alpha]_D^{22}$ = -68,1° (c=1, in Wasser).

d) H-Arg-Ile-Ser(tBu)-Arg-Pro-OtBu-triacetat

4,4, g Z-Arg-Ile-Ser(tBu)-Arg-Pro-OtBu-diacetat (Rohsubstanz) werden in ca. 60 ml 90 %iger Essigsäure gelöst und analog Beispiel 1b katalytisch hydriert.

Ausbeute 4,25 g.

1 g der oben gewonnenen Substanz wird an Kieselgel chromatographiert. Eluiert wird mit einer Mischung aus 450 ml Methylenchlorid, 200 ml Methanol, 150 ml Methylglycol, 100 ml Wasser und 5 g Ammoniumacetat, die man mit Eisessig auf pH 6 einstellt.

Ausbeute 560 mg, amorph, $[\alpha]_D^{22}$ = -53,6° (c=1, in Wasser).

Beispiel 26:

H-Arg-Trp-Asp(OtBu)-Arg-Phe-$NH_2$

a) Z-Arg($Z_2$)-Phe-$NH_2$•Dicyclohexylharnstoff

Zu einer Lösung von 8,65 g (15 mmol) Z-Arg($Z_2$)-OH, 3,01 g H-Phe-$NH_2$•HCl, 2,45 g HOObt und 1,95 ml N-Ethylmorpholin in 30 ml Dimethylformamid gibt man bei 0°C unter Rühren 3,3 g DCC. Man läßt 1 Stunde bei 0°C rühren und läßt dann bei Raumtemperatur stehen. Anderntags ist der Ansatz erstarrt. Man verrührt den Ansatz mit halbkonzentrierter $NaHCO_3$-Lösung, saugt ab und wäscht mit $KHSO_4$-Lösung und Wasser.

Ausbeute 13,9 g (Die Substanz enthält noch in etwa äquimolare Mengen an Dicyclohexylharnstoff), $[\alpha]_D^{20}$ = -2,3° (c=1, in 80 %iger Essigsäure).

b) H-Asp(OtBu)-Arg-Phe-$NH_2$•2 $HClO_4$

13,5 g Z-Arg($Z_2$)-Phe-$NH_2$•Dicyclohexylharnstoff werden in 200 ml Dimethylacetamid suspendiert und analog Beispiel 2c katalytisch hydriert. Man titriert hier mit 1N $HClO_4$. Der Rückstand wird in Wasser verrührt. Vom Unlöslichen wird abgesaugt und das Filtrat wird gefriergetrocknet. Das resultierende Öl wird 2 mal mit Diethylether und 2 mal mit Essigester verrieben und jeweils dekantiert. Der Rückstand wird im Hochvakuum getrocknet.

Ausbeute 9,5 g Öl.

Zu einer Lösung von oben gewonnenem 9,5 g Öl 5,9 g Z-Asp(OtBu)-OH, 2,48 g HOBt und 2,37 ml N-Ethylmorpholin in 50 ml Dimethylformamid gibt man bei 0°C unter Rühren 4 g DCC. Man arbeitet analog Beispiel 1a auf. Der Rückstand wird mit Diethylether verrieben und abgesaugt.

Ausbeute 9,00 g

8,7 g der oben gewonnenen Substanz [Z-Asp(OtBu)-Arg-Phe-$NH_2$•$HClO_4$] werden analog Beispiel 2c katalytisch hydriert. Man titriert hier mit 1N $HClO_4$.

Ausbeute 7,8 g, amorph, $[\alpha]_D^{20}$ = -4,0° (c=1, in Methanol).

c) Z-Trp-Asp(OtBu)-Arg-Phe-$NH_2$•$HClO_4$

Zu einer Lösung von 3,4 g (10 mmol) Z-Trp-OH, 6,92 g H-Asp(OtBu)-Arg-Phe-$NH_2$•2 $HClO_4$, 1,35 g HOBt und 1,3 ml N-Ethylmorpholin in 40 ml Dimethylformamid gibt man bei 0°C unter Rühren 2,2 g DCC. Man arbeitet analog Beispiel 1a auf.

Ausbeute 7,3 g, amorph, $[\alpha]_D^{21}$ = -17,5° (c=1, in Methanol).

d) H-Trp-Asp(OtBu)-Arg-Phe-$NH_2$•2 $HClO_4$

7 g Z-Trp-Asp(OtBu)-Arg-Phe-$NH_2$ werden analog Beispiel 2c katalytisch hydriert. Titriert wird mit 1N $HClO_4$.

Ausbeute 6,2 g, amorph, $[\alpha]_D^{20}$ = -12,6° (c=1, in Methanol).

e) Z-Arg($Z_2$)-Trp-Asp(OtBu)-Arg-Phe-$NH_2$•$HClO_4$

Zu einer Lösung von 577 mg (1 mmol) Z-Arg($Z_2$)-OH, 879 mg H-Trp-Asp(OtBu)-Arg-Phe-$NH_2$•2 $HClO_4$, 163 mg HOObt und 0,13 ml N-Ethylmorpholin in 10 ml Dimethylformamid gibt man bei 0°C unter Rühren 220 mg DCC. Man arbeitet analog Beispiel 25c auf. Hierbei fällt die Substanz zum größten Teil aus der n-Pentanolphase aus.

Ausbeute 1,16 g, $[\alpha]_D^{20}$ = -15,2° (c=1, in 80 %iger wäßriger Essigsäure).

f) H-Arg-Trp-Asp(OtBu)-Arg-Phe-$NH_2$-triacetat

3 g Z-Arg($Z_2$)-Trp-Asp(OtBu)-Arg-Phe-$NH_2$•$HClO_4$ werden analog Beispiel 1b katalytisch hydriert.

Ausbeute 2,33 g.

960 mg der oben gewonnenen Substanz werden analog Beispiel 25d chromatographisch gereinigt.

Ausbeute 540 mg, $[\alpha]_D^{22}$ = -14,2° (c=1, in Wasser).

Beispiel 27:

H-Lys-Phe-Leu-Lys-Phe-OH

a) Fmoc-Lys(Z)-Phe-OtBu

Zu einer Lösung von 5,02 g (10 mmol) Fmoc-Lys(Z)-OH, 2,6 g H-Phe-OtBu•HCl, 1,35 g HOBt und 1,3 ml N-Ethylmorpholin in 50 ml Dimethylformamid gibt man unter Rühren bei 0°C 2,2 g DCC. Man arbeitet analog Beispiel 1a auf.

Ausbeute 6,2 g, $[\alpha]_D^{22}$ = -6,7° (c=1, in 90 %iger Essigsäure).

b) Fmoc-Lys(Z)-Phe-OH

2,95 g Fmoc-Lys(Z)-Phe-OtBu werden in 30 ml einer 90 %igen wäßrigen Trifluoressigsäure gelöst. Man läßt eine Stunde bei Raumtemperatur stehen und engt im Vakuum ein. Der Rückstand wird mit Diethylether verrieben und abgesaugt.

Ausbeute 2,32 g, $[\alpha]_D^{20}$ = -2,3° (c=1, in Methanol).

c) Fmoc-Lys(Z)-Phe-Lys(Z)-Phe-OtBu

Zu einer Lösung von 3,17 g Fmoc-Lys(Z)-Phe-OtBu in 20 ml Dimethylformamid gibt man 4,7 ml Diethylamin. Man läßt 10 Minuten bei Raumtemperatur stehen und engt im Hochvakuum ein. Der Rückstand wird in Methylenchlorid gelöst, von Unlöslichem filtriert und über Kieselgel chromatographiert. Zunächst wird mit Methylenchlorid eluiert (Abtrennung der lipophilen Verunreinigung). Die Substanz wird mit einer Mischung aus Methylenchlorid/Methanol wie 95:5 eluiert.

Ausbeute 2,3 g.

Zu einer Lösung der oben gewonnenen 2,3 g H-Lys(Z)-Phe-OtBu, 1,58 g (4,5 mmol) Fmoc-Leu-OH und 607 mg HOBt in 20 ml Dimethylformamid gibt man bei 0°C 990 mg DCC. Man arbeitet analog Beispiel 1a auf.

Ausbeute 2,7 g.

Zu einer Lösung der oben gewonnenen 2,7 g Fmoc-Leu-Lys(Z)-Phe-OtBu in 20 ml Dimethylformamid gibt man 3,42 ml Diethylamin und arbeitet analog oben auf.

Ausbeute 1,95 g.

Zu einer Lösung der oben gewonnenen 1,95 g H-Leu-Lys(Z)-Phe-OtBu, 2,2 g Fmoc-Lys(Z)-Phe-OH und 533 mg HOObt in 20 ml Dimethylformamid gibt man bei 0°C und unter Rühren 720 mg DCC. Man läßt wie

üblich stehen, saugt den Niederschlag ab und engt das Filtrat ein. Der Rückstand wird mit gesättigter $NaHCO_3$-Lösung verrieben und abgesaugt.

Ausbeute 3,14 g, Schmp. 178-182 ° C, $[\alpha]_D^{22}$ = -20,8 ° (c = 1, in 90 %iger Essigsäure).

d) H-Lys-Phe-Leu-Lys-Phe-OtBu-triacetat

1 g Fmoc-Lys(Z)-Phe-Leu-Lys(Z)-Phe-OtBu werden analog Beispiel 1b katalytisch hydriert.

Ausbeute 750 mg.

Oben gewonnene Substanz wird analog Beispiel 25d chromatographisch gereinigt.

Ausbeute 450 mg, $[\alpha]_D^{23}$ = -25,5 ° (c = 1, in Wasser).

e) H-Lys-Phe-Leu-Lys-Phe-OH-diacetat

2,1 g Fmoc-Lys(Z)-Phe-Leu-Lys(Z)-Phe-OtBu werden in 21 ml 90 %iger wäßriger Trifluoressigsäure gelöst. Man läßt 1 Stunde bei Raumtemperatur stehen und engt im Vakuum ein. Der Rückstand wird mit Diethylether verrieben und abgesaugt.

Ausbeute 1,99 g.

1 g des oben gewonnenen Fmoc-Lys(Z)-Phe-Leu-Lys(Z)-Phe-OH werden analog Beispiel 1b katalytisch hydriert.

Ausbeute 630 mg.

Zur Reinigung werden oben gewonnene 630 mg analog Beispiel 25d chromatographisch gereinigt.

Ausbeute 458 mg, $[\alpha]_D^{23}$ = -9,7 ° (c = 1, in Wasser).

Beispiel 28:

H-Lys-Phe-Leu-Lys-Phe-$NH_2$-triacetat

Zu einer Lösung von 970 mg (0,83 mmol) Fmoc-Lys(Z)-Phe-Leu-Lys(Z)-Phe-OH (siehe Beispiel 27e) und 126 mg HOBt•$NH_3$ in 10 ml Dimethylformamid gibt man bei 0 ° C unter Rühren 182 mg DCC. Man arbeitet analog Beispiel 27c (unten) auf.

Ausbeute 950 mg.

Oben gewonnene 950 mg Fmoc-Lys(Z)-Phe-Leu-Lys(Z)-Phe-$NH_2$ werden analog Beispiel 1b katalytisch hydriert.

Ausbeute 635 mg.

Die oben gewonnenen 635 mg H-Lys-Phe-Leu-Lys-Phe-$NH_2$-triacetat werden analog Beispiel 25d chromatographisch gereinigt.

Ausbeute 298 mg, $[\alpha]_D^{23}$ = -21,4 ° (c = 1, in Wasser).

Beispiel 29:

H-Arg-Ile-Val-Arg-Ile-OH

a) Fmoc-Val-Arg-OH

Zu einer Suspension von 5,23 g (30 mmol) Arginin, 4,89 g HOObt und 5,4 g Pyridinperchlorat gibt man bei Raumtemperatur unter Rühren 14,55 g Fmoc-Val-OObt. Man läßt rühren bis alles gelöst ist und läßt über Nacht stehen. Die Lösung wird eingeengt und der Rückstand mit Wasser versetzt. Mit $NaHCO_3$-Lösung wird auf pH 7 gestellt. Der sich bildende Niederschlag wird abgesaugt und mit Wasser gewaschen.

Ausbeute 470 mg, $[\alpha]_D^{21}$ = -16,6 ° (c = 1, in Methanol).

b) Fmoc-Val-Arg-Ile-OBzl

Zu einer Lösung von 1,49 g (3 mmol) Fmoc-Val-Arg-OH, 1,18 g H-Ile-OBzl-tosylat und 489 mg HOObt in 15 ml Dimethylformamid gibt man bei 0 ° C und unter Rühren 660 mg DCC. Man arbeitet analog Beispiel 2b auf.

Ausbeute 2,2 g, $[\alpha]_D^{21}$ = -30,0 ° (c = 1, in Methanol).

c) Fmoc-Arg-Ile-OtBu

Zu einer Lösung von 2,23 g H-Ile-OtBu•HCl, 4g (10 mmol) Fmoc-Arg-OH und 1,35 g HOBt in 80 ml Dimethylformamid gibt man bei 0°C unter Rühren 2,2 g DCC. Man arbeitet analog Beispiel 2b auf.

Ausbeute 5,65 g, $[\alpha]_D^{21}$ = -13,8° (c=1, in Methanol).

d) Fmoc-Arg-Ile-OH

15,8 g Fmoc-Arg-Ile-OtBu werden in 150 ml 90 %iger wäßriger Trifluoressigsäure gelöst. Man läßt 1 Stunde bei Raumtemperatur stehen und engt ein. Der Rückstand wird 2 mal mit Diethylether verrieben, wobei der Ether jeweils dekantiert wird. Der Rückstand wird mit Wasser versetzt und mit gesättigter $NaHCO_3$-Lösung auf pH7 eingestellt. Der Niederschlag wird abgesaugt und getrocknet.

Ausbeute 9,8 g $[\alpha]_D^{21}$ = 5,3° (c=1, in Methanol).

e) H-Val-Arg-Ile-OBzl

Zu einer Lösung von 21 g (30 mmol) Fmoc-Val-Arg-Ile-OBzl in 200 ml Dimethylformamid gibt man 31,5 ml Diethylamin. Man läßt 10 Minuten bei Raumtemperatur stehen und engt im Vakuum ein. Der Rückstand wird mit Diethylether verrieben und abgesaugt.

Ausbeute 13,85 g, $[\alpha]_D^{21}$ = -27,2° (c=1, in Methanol).

f) Fmoc-Arg-Ile-Val-Arg-Ile-OBzl

Zu einer Lösung von 7,4 g (15 mmol) Fmoc-Arg-Ile-OH, 7,15 g H-Val-Arg-Ile-OBzl und 2,44 g HOObt in 150 ml Dimethylformamid gibt man bei 0°C unter Rühren 3,3 g DCC. Man arbeitet analog Beispiel 2b auf. Der getrocknete Rückstand wird mit Essigester verrieben, abgesaugt und getrocknet.

Ausbeute 14,1 g, $[\alpha]_D^{21}$ = -33,5° (c=1, in Methanol).

g) H-Arg-Ile-Val-Arg-Ile-OBzl-triacetat

Zu einer Lösung von 2,9 g (3 mmol) Fmoc-Arg-Ile-Val-Arg-Ile-OBzl in 20 ml Dimethylformamid gibt man 3,15 ml Diethylamin und läßt 10 Minuten bei Raumtemperatur stehen. Danach wird im Vakuum eingeengt und der Rückstand mit Diethylether verrieben.

Ausbeute 2,7 g.

800 mg der oben gewonnenen Substanz werden analog Beispiel 25d chromatographisch gereinigt.

Ausbeute 725 mg, $[\alpha]_D^{24}$ = -27,7° (c=1, in 90 %iger Essigsäure).

h) H-Arg-Ile-Val-Arg-Ile-OH-diacetat

1,5 g H-Arg-Ile-Val-Arg-Ile-OBzl (Rohsubstanz) werden analog Beispiel 1b katalytisch hydriert.

Ausbeute 1,3 g.

700 mg der oben gewonnenen Substanz werden analog Beispiel 25d chromatographisch gereinigt.

Ausbeute 405 mg, $[\alpha]_D^{22}$ = -47,1° (c=1, in Wasser).

Beispiel 30:

Cyclo-(D-Arg-Ile-D-Leu-Arg-Ile)-diacetat

1,4 g H-D-Arg-Ile-D-Leu-Arg-Ile-OH-diacetat(siehe Beispiel 13c) werden über einen schwach beasischen Ionenaustauscher in Perchlorat-Form chromatographiert. Das Eluat wird gefriergetrocknet.

Ausbeute 1,343 g H-D-Arg-Ile-D-Leu-Arg-Ile-OH-diperchlorat.

Zu einer Lösung von 440 mg DCC in 50 ml Dimethylformamid läßt man langsam eine Lösung aus 670 mg (1 mmol) des oben gewonnenen H-D-Arg-Ile-D-Leu-Arg-Ile-OH-diperchlorats, 270 mg HOBt und 0,13 ml N-Ethylmorpholin in 100 ml Dimethylformamid bei Raumtemperatur und unter Rühren zutropfen. Man läßt über Nacht bei Raumtemperatur stehen. Anderntags gibt man nochmals 300 mg DCC zu und läßt 2 Tage bei Raumtemperatur stehen. Danach wird im Vakuum eingeengt. Der Rückstand wird zwischen Essigester und verdünnter wäßriger Essigsäure verteilt. Die wäßrige Phase wird gefriergetrocknet.

Ausbeute 665 mg.

Oben gewonnene Substanz wird über einen schwach basischen Ionenaustauscher in Acetatform chromatographiert. Als Elutionsmittel dient Wasser. Das Peptid-haltige Eluat wird vereinigt und gefrierge-

trocknet.

Ausbeute 548 mg, $[\alpha]_D^{23}$ = -16,7° (c = 1, in Wasser).

Beispiel 31:

H-Arg-Pro-Cys(StBu)-Arg-Phe-OtBu

a) Fmoc-Arg-Pro-OtBu•$HClO_4$

Zu einer Lösung von 5,95 g (15 mmol) Fmoc-Arg-OH, 2,57 g H-Pro-OtBu, 2,02 g HOBt und 2,7 g Pyridiniumperchlorat in 30 ml Dimethylformamid gibt man bei 0°C unter Rühren 3,3 g DCC. Man arbeitet analog Beispiel 1a auf. Der Rückstand wird mit Diethylether verrieben und abgesaugt.

Ausbeute 9,4 g

Zur Charakterisierung werden 750 mg der oben gewonnenen Substanz in 40 %iger wäßriger Essigsäure gelöst und über einen schwach basischen Ionenaustauscher in Acetat-Form chromatographiert. Das Eluat wird eingeengt und gefriergetrocknet.

Ausbeute 690 mg Fmoc-Arg-Pro-OtBu-acetat, $C_{32}H_{43}N_5O_7$, (MG 609,7), $[\alpha]_D^{20}$ = -44,2° (c = 1, in Methanol), Gehalt an Substanz laut Aminosäureanalyse: 93 %.

b) Fmoc-Arg-Pro-OH

8,5 g Fmoc-Arg-Pro-OtBu•$HClO_4$ werden in 85 ml 90 %iger wäßriger Trifluoressigsäure gelöst. Man läßt 1 Stunde bei Raumtemperatur stehen und engt im Vakuum ein. Der Rückstand wird mit Diethylether verrieben und abgesaugt.

Ausbeute 7,9 g.

7,16 g der oben gewonnenen Substanz werden in 100 ml Wasser suspendiert. Mit einer gesättigten $NaHCO_3$-Lösung wird ein pH von 6 eingestellt. Man läßt 2 Tage bei 4°C stehen und saugt den Niederschlag ab und trocknet über $P_2O_5$ im Vakuum.

Ausbeute 6,15 g, $[\alpha]_D^{20}$ = -32,2° (c = 1, in 80 %iger wäßriger Essigsäure).

c) Z-Arg($Z_2$)-Phe-OtBu

Zu einer Lösung von 17,3 g (30 mmol) Z-Arg($Z_2$)-OH, 7,73 g H-Phe-OtBu•HCl, 4,9 g HOObt und 3,9 ml N-Ethylmorpholin in 70 ml Dimethylformamid gibt man unter Rühren bei 0°C 6,6 g DCC. Man arbeitet analog Beispiel 1a auf. Der Rückstand wird mit Diethylether verrieben, abgesaugt und im Vakuum getrocknet.

Ausbeute 19 g, $[\alpha]_D^{20}$ = -0,5° (c = 1, in 80 %iger wäßriger Essigsäure).

d) H-Arg-Phe-OtBu•$HClO_4$

18,6 g Z-Arg($Z_2$)-Phe-OtBu werden in 200 ml Dimethylacetamid gelöst und über Pd/C katalytisch mit Wasserstoff hydriert. Mit Hilfe eines Autotitrators wird während der Hydrierung durch Zusatz von 1N $HClO_4$ pH 5 gehalten. Nach beendigter Hydrierung wird der Katalysator abgesaugt und das Filtrat eingeengt. Der Rückstand wird in Wasser gelöst, über eine Klärschicht filtriert und gefriergetrocknet.

Ausbeute 21,1 g Öl.

Das Öl wird zweimal mit Diethylether verrieben, der Diethylether abdekantiert und der ölige Rückstand im Vakuum getrocknet.

Ausbeute 18,5 g (135 %, enthält also noch Lösungsmittel).

e) Fmoc-Cys(StBu)-Arg-Phe-OtBu-acetat

Zu einer Lösung von 4,31 g (10 mmol) Fmoc-Cys(StBu)-OH, 7,74 g H-Arg-Phe-OtBu•2 $HClO_4$ (oben gewonnene ölige Substanz), 1,35 g HOBt und 1,3 ml N-Ethylmorpholin in 40 ml Dimethylformamid gibt man bei 0°C unter Rühren 2,2 g DCC. Der Ansatz wird analog Beispiel 1a aufgearbeitet. Der Rückstand wird mit Diethylether verrieben und getrocknet.

Ausbeute 8 g.

1,3 g der oben gewonnenen Substanz werden in 30 ml 55 %iger Essigsäure gelöst und über einen schwach basischen Ionenaustauscher in Acetat-Form chromatographiert. Man eluiert mit 55 %iger Essigsäure. Das Eluat wird eingeengt, in Wasser aufgenommen und gefriergetrocknet.

Ausbeute 1,05 g, $[\alpha]_D^{29}$ = -45,5° (c=1, in Methanol), Gehalt an Peptidbase laut Aminosäureanalyse: 79%

f) H-Cys(StBu)-Arg-Phe-OtBu•$HClO_4$

Zu einer Lösung von 17,84 g (20 mmol) Fmoc-Cys(StBu)-Arg-Phe-OtBu•$HClO_4$ (Rohsubstanz von Beispiel 31e) in 100 ml Dimethylformamid gibt man bei Raumtemperatur 21 ml (200 mmol) Diethylamin. Man rührt 10 Minuten bei Raumtemperatur und engt im Vakuum ein. Der Rückstand wird zweimal mit Diethylether verrieben und dekantiert. Der ölige Rückstand wird im Hochvakuum getrocknet.

Ausbeute 14,6 g.

g) Fmoc-Arg-Pro-Cys(StBu)-Arg-Phe-OtBu-diacetat

Zu einer Lösung von 4,94 g (10 mmol) Fmoc-Arg-Pro-OH, 5,7 g (10 mmol) H-Cys(StBu)-Arg-Phe-OtBu•$HClO_4$, 163 mg HOObt und 1,8 g Pyridiniumperchlorat in 100 ml Dimethylformamid gibt man bei 0°C 2,2 g DCC. Man arbeitet analog Beispiel 1a auf. Der Rückstand wird mit Diethylether verrieben.

Ausbeute 8,7 g.

500 mg der oben gewonnenen Substanz werden analog Beispiel 25d chromatographisch gereinigt.

Ausbeute 341 mg, $[\alpha]_D^{22}$ = -60,8° (c=1, Wasser), Gehalt an Peptidbase laut Aminosäureanalyse: 77 %.

h) H-Arg-Pro-Cys(StBu)-Arg-Phe-OtBu-triacetat

Zu einer Lösung von 3,3 g Fmoc-Arg-Pro-Cys(StBu)-Arg-Phe-OtBu (Rohsubstanz) in 20 ml Dimethylformamid gibt man 3,32 ml Diethylamin. Nach 10 Minuten bei Raumtemperatur engt man im Vakuum ein. Der Rückstand wird mit Ether verrieben und abgesaugt.

Ausbeute 2,7 g.

Die Substanz wird analog Beispiel 25d chromatographisch gereinigt.

Ausbeute 1,23 g, $[\alpha]_D^{22}$ = -42,4° (c=1, in Wasser),
Gehalt an Peptidbase laut Aminosäureanalyse: 72 %.

Beispiel 32:

Cyclo-(D-Arg-Ile-D-Leu-Arg-D-Val)

a) Fmoc-D-Leu-Arg-OH

Zu einer Suspension von 3,48 g (20 mmol) Arginin, 3,26 g HOObt und 3,6 g Pyridiniumperchlorat in 100 ml Dimethylformamid gibt man bei Raumtemperatur unter Rühren 9,97 g Fmoc-D-Leu-OObt. Man rührt bis alles gelöst ist und läßt über Nacht stehen. Danach engt man ein und versetzt den Rückstand mit Wasser. Durch Zugabe von $NaHCO_3$-Lösung wird ein pH von 7 eingestellt. Der Niederschlag wird abgesaugt und getrocknet.

Ausbeute 10,2 g, $[\alpha]_D^{20}$ = +29,2° (c=1, in Methanol).

b) Fmoc-D-Leu-Arg-D-Val-OBzl

Zu einer Lösung von 7,64 g (15 mmol) Fmoc-D-Leu-Arg-OH, 5,7 g H-D-Val-OBzl-tosylat und 2,45 g HOObt in 75 ml Dimethylformamid gibt man bei 0°C unter Rühren 3,3 g DCC. Man verfährt wie üblich und verteilt den Rückstand zwischen Essigester und gesättigter $NaHCO_3$-Lösung. Die Essigesterlösung wird noch mit Wasser ausgeschüttelt, über $Na_2SO_4$ getrocknet und eingeengt.

Ausbeute 8,2 g, $[\alpha]_D^{20}$ = +11,2° (c=1, in Methanol).

c) H-D-Leu-Arg-D-Val-OBzl

Zu einer Lösung von 8,04 g (11,5 mmol) Fmoc-D-Leu-Arg-D-Val-OBzl in 60 ml dimethylformamid gibt man 12,05 ml Diethylamin. Man läßt 15 Minuten bei Raumtemperatur stehen und engt ein. Der Rückstand wird zwischen n-Pentanol und $NaHCO_3$-Lösung verteilt. Die Pentanolphase wird eingeengt und der Rückstand mit Diethylether verrieben.

Ausbeute 3,8 g, $[\alpha]_D^{20}$ = -3,0° (c=1, in Methanol)

d) Z-D-Arg-Ile-D-Leu-Arg-D-Val-OBzl-diacetat

Zu einer Lösung von 3,16g Z-D-Arg-Ile-OH (siehe Beispiel 9b), 3,57 g H-D-Leu-Arg-D-Val-OBzl, 1,23 g HOObt und 1,35 g Pyridiniumperchlorat in 75 ml Dimethylformamid gibt man bei 0°C unter Rühren 1,65 g DCC. Man arbeitet wie üblich auf und verteilt den Rückstand zwischen n-Pentanol und halbgesättigter $NaHCO_3$-Lösung. Die n-Pentanol-Phase wird eingeengt und der Rückstand mit Essigester verrieben und abgesaugt. Die Substanz wird zwischen Essigester und 10 %iger wäßriger Essigsäure in 5 Stufen gegenstromverteilt. Die Verbindung ist in den Essigsäure-Phasen. Sie werden vereinigt und eingeengt. Der Rückstand wird mit Diethylether verrieben.

Ausbeute 4,55 g, $[\alpha]_D^{25}$ = +11,4° (c=1, in Methanol).

e) H-D-Arg-Ile-D-Leu-Arg-D-Val-OH-diacetat

4,4 g Z-D-Arg-Ile-D-Leu-Arg-D-Val-OBzl werden analog Beispiel 1b katalytisch hydriert.
Ausbeute 3,7 g.
700 mg der oben gewonnenen Verbindung werden analog Beispiel 25d chromatographisch gereinigt.
Ausbeute 350 mg, $[\alpha]_D^{22}$ = -10,6° (c=1, in Wasser).

f) cyclo-(D-Arg-Ile-D-Leu-Arg-D-Val)-diacetat

2 g H-D-Arg-Ile-D-Leu-Arg-D-Val-OH-diacetat (Rohsubstanz) werden analog Beispiel 30 in das entsprechende Di-perchlorat überführt.
Ausbeute 1,78 g.
656 mg (1 mmol) von oben gewonnener Substanz werden analog Beispiel 30 cyclisiert.
Ausbeute 683 mg.
Oben gewonnene Substanz wird analog Beispiel 25d chromatographisch gereinigt.
Ausbeute 366 mg, $[\alpha]_D^{21}$ = +20,5° (c=1, in Wasser).

Verwendete Abkürzungen:

Die für Aminosäuren verwendeten Abkürzungen entsprechen dem in der Peptidchemie üblichen Drei-Buchstaben-Code wie er z.B. in Europ. J. Biochem. 138, 9 (1984) beschrieben ist. Einige weitere Abkürzungen sind nachfolgend aufgelistet:

| | |
|---|---|
| Boc | tert.-Butyloxycarbonyl |
| Bzl | Benzyl |
| Fmoc | 9-Fluorenylmethyloxycarbonyl |
| Me | Methyl |
| Ac | Acetyl |
| DCC | Dicyclohexylcarbodiimid |
| FAB | Fast atom bombardment |
| HOBt | 1-Hydroxybenzotriazol |
| M | Molekularpeak |
| Z | Benzyloxycarbonyl |
| NSu | Succinimido |
| Tcp | 2,4,5-Trichlorphenyl |
| HOObt | 3-Hydroxy-4-oxo-3,4-dihydro-benzo[d]-1,2,3-triazin |
| DCHA | Dicyclohexylamin |

**Patentansprüche**
**Patentansprüche für folgende Vertragsstaaten : BE, CH, DE, FR, GB, IT, LI, LU, NL, SE**

**1.** Verbindung der Formel I

$R^N$-L-N-$B^2$-$R^C$ (I)

in welcher

$R^N$ für einen Rest der Formel II steht;

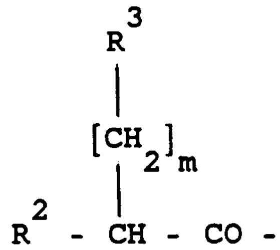

(II)

$R^2$ für Wasserstoff oder einen Rest der Formel $R-[A]_n-NH-$ steht;

$R^3$ Amino, Guanidino, $(C_1-C_3)$-Alkylamino oder Di-$(C_1-C_3)$-alkylamino bedeutet;

m eine ganze Zahl von 1-6 bedeutet;

A für einen Rest der Formel $-NH-CR^4R^5-CO-$ steht;

R Wasserstoff, $(C_1-C_6)$-Alkanoyl, $(C_7-C_{11})$-Aroyl, das im aromatischen Teil gegebenenfalls durch $(C_1-C_4)$-Alkyl, $(C_1-C_4)$-Alkoxy, $(C_1-C_4)$-Alkylthio, Halogen, Carbamoyl, $(C_1-C_4)$-Alkoxycarbonyl und/oder Sulfamoyl mono- oder disubstituiert oder durch Methylendioxy monosubstituiert ist, $(C_5-C_7)$-Cycloalkyl-$(C_1-C_3)$-alkanoyl oder $(C_6-C_{14})$-Aryl-$(C_1-C_3)$-alkanoyl bedeutet,
wobei in den Resten R $\neq$ Wasserstoff eine $-CH_2$-Gruppe gegebenenfalls durch -O- oder -S- ersetzt ist;

n 0 oder 1 ist;

$R^4$ und $R^5$ gleich oder verschieden sind und Wasserstoff, $(C_1-C_6)$-Alkyl oder $(C_7-C_{11})$-Aralkanoyl bedeuten;

L für Pro, D-Pro oder einen Rest der Formel $-NH-CH(R^6)-CO-$ steht;

$R^6$ $(C_1-C_6)$-Alkyl, das gegebenenfalls durch Hydroxy, $(C_1-C_6)$-Alkoxy, $(C_1-C_6)$-Alkoxycarbonyl, Carbamoyl oder R-NH monosubstituiert ist, wobei R wie oben definiert ist, aber nicht Wasserstoff sein kann, oder $(C_7-C_{11})$-Aralkyl, das am Aromaten gegebenenfalls durch $(C_1-C_6)$-Alkoxy monosubstituiert ist, oder Indol-3-ylmethyl bedeutet;

N für Pro, D-Pro oder einen Rest der Formel $-NH-CH(R^7)-CO-$ steht;

$R^7$ $(C_1-C_6)$-Alkyl, das gegebenenfalls durch Hydroxy, $(C_1-C_6)$-Alkoxy, $(C_1-C_6)$-Alkoxycarbonyl, Carbamoyl oder R-NH monosubstituiert ist, wobei R wie oben definiert ist, aber nicht Wasserstoff sein kann, oder $(C_7-C_{11})$-Aralkyl, das am Aromaten gegebenenfalls durch $(C_1-C_6)$-Alkoxy monosubstituiert ist, oder Indol-3-ylmethyl bedeutet;

$B^2$ für einen Rest der Formel III

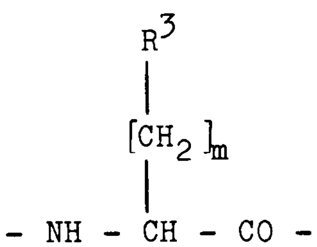

(III)

worin $R^3$ und m wie oben definiert sind, steht;

$R^C$ für eine Rest der Formel $-NR^9-CH(R^8)-(CO)_p-R^1$ steht;

$R^8$ wie $R^7$ definiert ist, wobei vorhandene CH-, $CH_2$- oder $CH_3$-Reste in $\beta$-Stellung bezüglich -NH- gegebenenfalls monohydroxyliert sind, und

$R^9$ Wasserstoff bedeutet;

oder

$R^8$ und $R^9$ zusammen $-[CH_2]_3-$ oder $-[CH_2]_4-$ bedeuten;

p 0 oder 1 ist;

$R^1$ im Falle von p = 0 für Wasserstoff, Hydroxy oder $(C_1-C_6)$-Alkoxy steht;

$R^1$ im Falle von p = 1 für $OR^{10}$ oder $NR^{10}R^{11}$ steht;
und
$R^{10}$ und $R^{11}$ gleich oder verschieden sind und Wasserstoff, $(C_1\text{-}C_6)$-Alkyl oder $(C_7\text{-}C_{11})$-Aralkyl bedeuten; oder $NR_{10}R_{11}$ für Pyrrolidino, Piperidino oder Morpholino steht;
oder
p = 1 ist,
R und $R^1$ gemeinsam eine Peptidbindung bedeuten und die übrigen Reste wie oben definiert sind,

sowie deren physiologisch verträglichen Salze.

**2.** Verbindung der Formel I gemäß Anspruch 1,
in welcher
L für den Rest von Isoleucin, Valin, Threonin, Serin, O-$(C_1\text{-}C_9)$-Alkylthreonin, O-$(C_1\text{-}C_9)$-Alkylserin, Leucin, Prolin oder des $\omega$-$(C_1\text{-}C_6)$-Alkyl- vorzugsweise tert. Butylesters von Glutaminsäure oder Asparaginsäure steht;
N für den Rest von Valin, Isoleucin, Leucin, Phenylalanin, Tryptophan, gegebenenfalls O-$(C_1\text{-}C_6)$-alkyliertem Tyrosin, Glutamin, Asparagin, Glutaminsäure-$\gamma$-$(C_1\text{-}C_6)$-alkylester oder Asparaginsäure-$\beta$-$(C_1\text{-}C_6)$-alkylester oder $\epsilon$-Acyl-lysin steht,
und
$B^2$ Arg, D-Arg, Lys oder D-Lys bedeutet,

sowie deren physiologisch verträglichen Salze.

**3.** Verbindung der Formel I gemäß Anspruch 1 oder 2,
in welcher p = 1 ist und R und $R^1$ gemeinsam für eine Peptidbindung stehen, sowie deren physiologisch verträglichen Salze.

**4.** Verbindung der Formel I gemäß Anspruch 1 oder 2,
in welcher R und $R^1$ nicht gemeinsam für eine Peptidbindung stehen,
sowie deren physiologisch verträglichen Salze.

**5.** Verfahren zur Herstellung einer Verbindung der Formel I gemäß einem der Ansprüche 1 - 4, dadurch gekennzeichnet, daß man ein Fragment mit endständiger Carboxylgruppe oder reaktives Derivat mit einem entsprechenden Fragment mit freier Aminogruppe kuppelt, gegebenenfalls zum Schutz weiterer funktioneller Gruppen (eine) temporär eingeführte Schutzgruppe(n) abspaltet und die so erhaltene Verbindung gegebenenfalls in ihr physiologisch verträgliches Salz überführt.

**6.** Verbindung der Formel I gemäß einem der Ansprüche 1 - 4 zur Anwendung als Heilmittel.

**7.** Verbindung der Formel I gemäß einem der Ansprüche 1 - 4 zur Anwendung als Heilmittel mit diuretischer und natriuretischer Wirkung.

**8.** Pharmazeutisches Mittel enthaltend eine Verbindung der Formel I gemäß einem der Ansprüche 1 - 4.

**9.** Verfahren zur Herstellung eines pharmazeutischen Mittels gemäß Anspruch 8, dadurch gekennzeichnet, daß man eine Verbindung gemäß einem der Ansprüche 1 - 4 zusammen mit einem Träger und gegebenenfalls weiteren Zusatz- und Hilfsstoffen in eine geeignete Darreichungsform bringt.

**Patentansprüche für folgende Vertragsstaaten : AT, ES, GR**

**1.** Verfahren zur Herstellung einer Verbindung der Formel I

$R^N$-L-N-$B^2$-$R^C$ (I)

in welcher
$R^N$ für einen Rest der Formel II steht;

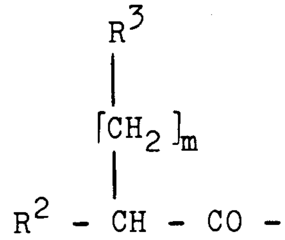

(II)

$R^2$ für Wasserstoff oder einen Rest der Formel $R-[A]_n-NH-$ steht;

$R^3$ Amino, Guanidino, $(C_1-C_3)$-Alkylamino oder Di-$(C_1-C_3)$-alkylamino bedeutet;

m eine ganze Zahl von 1-6 bedeutet;

A für einen Rest der Formel $-NH-CR^4R^5-CO-$ steht;

R Wasserstoff, $(C_1-C_6)$-Alkanoyl, $(C_7-C_{11})$-Aroyl, das im aromatischen Teil gegebenenfalls durch $(C_1-C_4)$-Alkyl, $(C_1-C_4)$-Alkoxy, $(C_1-C_4)$-Alkylthio, Halogen, Carbamoyl, $(C_1-C_4)$-Alkoxycarbonoyl und/oder Sulfamoyl mono- oder disubstituiert oder durch Methylendioxy monosubstituiert ist, $(C_5-C_7)$-Cycloalkyl-$(C_1-C_3)$-alkanoyl oder $(C_6-C_{14})$-Aryl-$(C_1-C_3)$-alkanoyl bedeutet,
wobei in den Resten R ≠ Wasserstoff eine $-CH_2$-Gruppe gegebenenfalls durch -O- oder -S- ersetzt ist;

n 0 oder 1 ist;

$R^4$ und $R^5$ gleich oder verschieden sind und Wasserstoff, $(C_1-C_6)$-Alkyl oder $(C_7-C_{11})$-Aralkanoyl bedeuten;

L für Pro, D-Pro oder einen Rest der Formel $-NH-CH(R^6)-CO-$ steht;

$R_6$ $(C_1-C_6)$-Alkyl, das gegebenenfalls durch Hydroxy, $(C_1-C_6)$-Alkoxy, $(C_1-C_6)$-Alkoxycarbonyl, Carbamoyl oder R-NH monosubstituiert ist, wobei R wie oben definiert ist, aber nicht Wasserstoff sein kann, oder $(C_7-C_{11})$-Aralkyl, das am Aromaten gegebenenfalls durch $(C_1-C_6)$-Alkoxy monosubstituiert ist, oder Indol-3-ylmethyl bedeutet;

N für Pro, D-Pro oder einen Rest der Formel $-NH-CH(R^7)-CO-$ steht;

$R^7$ $(C_1-C_6)$-Alkyl, das gegebenenfalls durch Hydroxy, $(C_1-C_6)$-Alkoxy, $(C_1-C_6)$-Alkoxycarbonyl, Carbamoyl oder R-NH monosubstituiert ist, wobei R wie oben definiert ist, aber nicht Wasserstoff sein kann, oder $(C_7-C_{11})$-Aralkyl, das am Aromaten gegebenenfalls durch $(C_1-C_6)$-Alkoxy monosubstituiert ist, oder Indol-3-ylmethyl bedeutet;

$B^2$ für einen Rest der Formel III

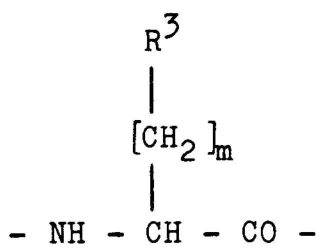

(III)

worin $R^3$ und m wie oben definiert sind, steht;

$R^C$ für eine Rest der Formel $-NR^9-CH(R^8)-(CO)_p-R^1$ steht;

$R^8$ wie $R^7$ definiert ist, wobei vorhandene CH-, $CH_2$- oder $CH_3$-Reste in β-Stellung bezüglich -NH- gegebenenfalls monohydroxyliert sind, und

$R^9$ Wasserstoff bedeutet;
oder

$R^8$ und $R^9$ zusammen $-[CH_2]_3-$ oder $-[CH_2]_4-$ bedeuten;

p 0 oder 1 ist;

$R^1$ im Falle von p = 0 für Wasserstoff, Hydroxy oder $(C_1-C_6)$-Alkoxy steht;

$R^1$ im Falle von p = 1 für $OR^{10}$ oder $NR^{10}R^{11}$ steht;
und

$R^{10}$ und $R^{11}$ gleich oder verschieden sind und Wasserstoff, $(C_1-C_6)$-Alkyl oder $(C_7-C_{11})$-Aralkyl

bedeuten; oder $NR^{10}R^{11}$ für Pyrrolidino, Piperidino oder Morpholino steht;
oder

p = 1 ist,

R und $R^1$ gemeinsam eine Peptidbindung bedeuten und die übrigen Reste wie oben definiert sind,

sowie deren physiologisch verträglichen Salze, dadurch gekennzeichnet, daß man ein Fragment mit endständiger Carboxylgruppe oder reaktives Derivat mit einem entsprechenden Fragment mit freier Aminogruppe kuppelt, gegebenenfalls zum Schutz weiterer funktioneller Gruppen (eine) temporär eingeführte Schutzgruppe(n) abspaltet und die so erhaltene Verbindung gegebenenfalls in ihr physiologisch verträgliches Salz überführt.

**2.** Verfahren gemäß Anspruch 1, dadurch gekennzeichnet, daß man eine Verbindung der Formel I herstellt, in welcher

L für den Rest von Isoleucin, Valin, Threonin, Serin, O-($C_1$-$C_9$)-Alkylthreonin, O-($C_1$-$C_9$)-Alkylserin, Leucin, Prolin oder des $\omega$-($C_1$-$C_6$)-Alkyl- vorzugsweise tert. Butylesters von Glutaminsäure oder Asparaginsäure steht;

N für den Rest von Valin, Isoleucin, Leucin, Phenylalanin, Tryptophan, gegebenenfalls O-($C_1$-$C_6$)-alkyliertem Tyrosin, Glutamin, Asparagin, Glutaminsäure-$\gamma$-($C_1$-$C_6$)-alkylester oder Asparaginsäure-$\beta$-($C_1$-$C_6$)-alkylester oder $\epsilon$-Acyl-lysin steht,
und

$B^2$ Arg, D-Arg, Lys oder D-Lys bedeutet,

sowie deren physiologisch verträglichen Salze.

**3.** Verfahren gemäß Anspruch 1 oder 2, dadurch gekennzeichnet, daß man eine Verbindung der Formel I herstellt,
in welcher p = 1 ist und R und $R^1$ gemeinsam für eine Peptidbindung stehen, sowie deren physiologisch verträglichen Salze.

**4.** Verfahren gemäß einem der Ansprüche 1 bis 3, dadurch gekennzeichnet, daß man eine Verbindung herstellt,
in welcher R und $R^1$ nicht gemeinsam für eine Peptidbindung stehen,
sowie deren physiologisch verträglichen Salze.

**5.** Verfahren zur Herstellung eines pharmazeutischen Mittels enthaltend eine Verbindung der Formel I hergestellt gemäß einem der Ansprüche 1 - 4, dadurch gekennzeichnet, daß man diese zusammen mit einem Träger und gegebenenfalls weiteren Zusatz- und Hilfsstoffen in eine geeignete Darreichungsform bringt.

## Claims

## Claims for the following Contracting States : BE, CH, DE, FR, GB, IT, LI, LU, NL, SE

**1.** A compound of the formula I

$R^N$-L-N-$B^2$-$R^C$ (I)

in which

$R^N$ represents a radical of the formula II;

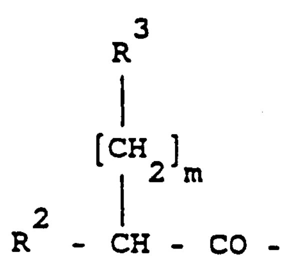

(II)

$R^2$ represents hydrogen or a radical of the formula $R-[A]_n-NH-$;

$R^3$ denotes amino, guanidino, $(C_1-C_3)$-alkylamino or di-$(C_1-C_3)$-alkylamino;

m denotes an integer from 1 to 6;

A represents a radical of the formula $-NH-CR^4R^5-CO-$;

R denotes hydrogen, $(C_1-C_6)$-alkanoyl, $(C_7-C_{11})$-aroyl, in which the aromatic moiety is optionally mono- or disubstituted by $(C_1-C_4)$-alkyl, $(C_1-C_4)$-alkoxy, $(C_1-C_4)$-alkylthio, halogen, carbamoyl, $(C_1-C_4)$-alkoxycarbonyl and/or sulfamoyl, or is monosubstituted by methylenedioxy, or denotes $(C_5-C_7)$-cyclo-alkyl-$(C_1-C_3)$-alkanoyl or $(C_6-C_{14})$-aryl-$(C_1-C_3)$-alkanoyl, where a $-CH_2$ group in the radicals R $\neq$ hydrogen is optionally replaced by -O- or -S-;

n is 0 or 1;

$R^4$ and $R^5$ are identical or different and denote hydrogen, $(C_1-C_6)$-alkyl or $(C_7-C_{11})$-aralkanoyl;

L represents Pro, D-Pro or a radical of the formula $-NH-CH(R^6)-CO-$;

$R^6$ denotes $(C_1-C_6)$-alkyl which is optionally monosubstituted by hydroxyl, $(C_1-C_6)$-alkoxy, $(C_1-C_6)$-alkoxycarbonyl, carbamoyl or R-NH, where R is as defined above but cannot be hydrogen, or denotes $(C_7-C_{11})$-aralkyl which is optionally monosubstituted on the aromatic ring by $(C_1-C_6)$-alkoxy, or denotes 3-indolylmethyl;

N represents Pro, D-Pro or a radical of the formula $-NH-CH(R^7)-CO-$;

$R^7$ denotes $(C_1-C_6)$-alkyl which is optionally monosubstituted by hydroxyl, $(C_1-C_6)$-alkoxy, $(C_1-C_6)$-alkoxycarbonyl, carbamoyl or R-NH, where R is as defined above but cannot be hydrogen, or denotes $(C_7-C_{11})$-aralkyl which is optionally monosubstituted on the aromatic ring by $(C_1-C_6)$-alkoxy, or denotes 3-indolylmethyl;

$B^2$ represents a radical of the formula III

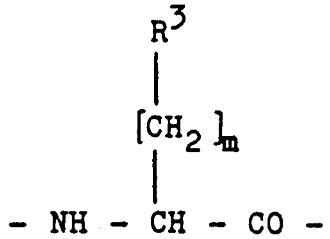

(III)

in which $R^3$ and m are as defined above;

$R^C$ represents a radical of the formula $-NR^9-CH(R^8)-(CO)_p-R^1$;

$R^8$ is defined as $R^7$, with CH, $CH_2$ or $CH_3$ radicals which are present in the $\beta$-position with respect to -NH- optionally being monohydroxylated, and

$R^9$ denotes hydrogen; or

$R^8$ and $R^9$ together denote $-[CH_2]_3-$ or $-[CH_2]_4-$;

P is 0 or 1;

$R^1$ represents hydrogen, hydroxyl or $(C_1-C_6)$-alkoxy in the case of p = 0;

$R^1$ represents $OR^{10}$ or $NR^{10}R^{11}$ in the case of p = 1; and

$R^{10}$ and $R^{11}$ are identical or different and denote hydrogen, $(C_1-C_6)$-alkyl or $(C_7-C_{11})$-aralkyl; or $NR^{10}R^{11}$ represents pyrrolidino, piperidino or morpholino; or

p is 1,

R and $R^1$ together denote a peptide linkage, and the other radicals are as defined above,

and its physiologically tolerated salts.

**2.** A compound of the formula I as claimed in claim 1, in which

L represents the radical of isoleucine, valine, threonine, serine, O-$(C_1-C_9)$-alkylthreonine, O-$(C_1-C_9)$-alkylserine, leucine, proline or of the $\omega$-$(C_1-C_6)$-alkyl ester, preferably tert. butyl ester, of glutamic acid or aspartic acid;

N represents the radical of valine, isoleucine, leucine, phenylalanine, tryptophan, tyrosine which is optionally O-$(C_1-C_6)$-alkylated, glutamine, asparagine, $\gamma$-$(C_1-C_6)$-alkyl glutamate or $\beta$-$(C_1-C_6)$-alkyl aspartate or $\epsilon$-acyl-lysine, and

$B^2$ denotes Arg, D-Arg, Lys or D-Lys,

and its physiolgically tolerated salts.

**3.** A compound of the formula I as claimed in claim 1 or 2, in which p is 1, and R and $R^1$ together represent a peptide linkage, and its physiologically tolerated salts.

**4.** A compound of the formula I as claimed in claim 1 or 2, in which R and $R^1$ do not together represent a peptide linkage, and its physiologically tolerated salts.

**5.** A process for the preparation of a compound of the formula I as claimed in one of claims 1-4, which comprises coupling a fragment with a terminal carboxyl group, or its reactive derivative, with an appropriate fragment with a free amino group, where appropriate eliminating (a) protective group(s) temporarily introduced to protect other functional groups, and converting, where appropriate, the compound which has thus been obtained into its physiologically tolerated salt.

**6.** A compound of the formula I as claimed in one of claims 1-4 for use as medicine.

**7.** A compound of the formula I as claimed in one of claims 1-4 for use as medicine having diuretic and natriuretic actions.

**8.** A pharmaceutical agent containing a compound of the formula I as claimed in one of claims 1-4.

**9.** A process for the preparation of a pharmaceutical agent as claimed in claim 8, which comprises converting a compound as claimed in one of claims 1-4, together with a vehicle and, where appropriate, other additives and auxiliaries, into a suitable form for administration.

**Claims for the following Contracting States : AT, ES, GR**

**1.** A process for the preparation of a compound of the formula I

$R^N$-L-N-$B^2$-$R^C$ (I)

in which

$R^N$ represents a radical of the formula II;

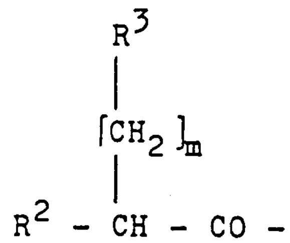

(II)

| | |
|---|---|
| $R^2$ | represents hydrogen or a radical of the formula R-$[A]_n$-NH-; |
| $R^3$ | denotes amino, guanidino, $(C_1$-$C_3)$-alkylamino or di-$(C_1$-$C_3)$-alkylamino; |
| m | denotes an integer from 1 to 6; |
| A | represents a radical of the formula -NH-$CR^4R^5$-CO-; |
| R | denotes hydrogen, $(C_1$-$C_6)$-alkanoyl, $(C_7$-$C_{11})$-aroyl, in which the aromatic moiety is optionally mono- or disubstituted by $(C_1$-$C_4)$-alkyl, $(C_1$-$C_4)$-alkoxy, $(C_1$-$C_4)$-alkylthio, halogen, carbamoyl, $(C_1$-$C_4)$-alkoxycarbonyl and/or sulfamoyl, or is monosubstituted by methylenedioxy, or denotes $(C_5$-$C_7)$-cyclo-alkyl-$(C_1$-$C_3)$-alkanoyl or $(C_6$-$C_{14})$-aryl-$(C_1$-$C_3)$-alkanoyl, where a -$CH_2$ group in the radicals R ≠ hydrogen is optionally replaced by -O- or -S-; |
| n | is 0 or 1; |
| $R^4$ and $R^5$ | are identical or different and denote hydrogen, $(C_1$-$C_6)$-alkyl or $(C_7$-$C_{11})$-aralkanoyl; |
| L | represents Pro, D-Pro or a radical of the formula -NH-CH$(R^6)$-CO-; |
| $R^6$ | denotes $(C_1$-$C_6)$-alkyl which is optionally monosubstituted by hydroxyl, $(C_1$-$C_6)$-alkoxy, $(C_1$-$C_6)$-alkoxycarbonyl, carbamoyl or R-NH, where R is as defined above but cannot be hydrogen, or denotes $(C_7$-$C_{11})$-aralkyl which is optionally monosub- |

stituted on the aromatic ring by ($C_1$-$C_6$)-alkoxy, or denotes 3-indolylmethyl;

N represents Pro, D-Pro or a radical of the formula -NH-CH($R^7$)-CO-;

$R^7$ denotes ($C_1$-$C_6$)-alkyl which is optionally monosubstituted by hydroxyl, ($C_1$-$C_6$)-alkoxy, ($C_1$-$C_6$)-alkoxycarbonyl, carbamoyl or R-NH, where R is as defined above but cannot be hydrogen, or denotes ($C_7$-$C_{11}$)-aralkyl which is optionally monosubstituted on the aromatic ring by ($C_1$-$C_6$)-alkoxy, or denotes 3-indolylmethyl;

$B^2$ represents a radical of the formula III

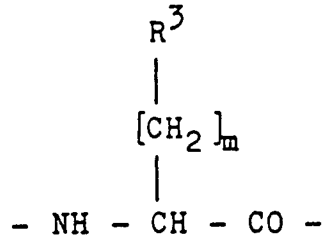

(III)

in which $R^3$ and m are as defined above;

$R^C$ represents a radical of the formula -$NR^9$-CH($R^8$)-$(CO)_p$-$R^1$;

$R^8$ is defined as $R^7$, with CH, $CH_2$ or $CH_3$ radicals which are present in the $\beta$-position with respect to -NH- optionally being monohydroxylated, and

$R^9$ denotes hydrogen; or

$R^8$ and $R^9$ together denote -$[CH_2]_3$- or -$[CH_2]_4$-;

p is 0 or 1;

$R^1$ represents hydrogen, hydroxyl or ($C_1$-$C_6$)-alkoxy in the case of p = 0;

$R^1$ represents $OR^{10}$ or $NR^{10}R^{11}$ in the case of p = 1; and

$R^{10}$ and $R^{11}$ are identical or different and denote hydrogen, ($C_1$-$C_6$)-alkyl or ($C_7$-$C_{11}$)-aralkyl; or $NR^{10}R^{11}$ represents pyrrolidino, piperidino or morpholino; or

p is 1,

R and $R^1$ together denote a peptide linkage, and the other radicals are as defined above,

and its physiologically tolerated salts, which comprises coupling a fragment with a terminal carboxyl group, or its reactive derivative, with an appropriate fragment with a free amino group, where appropriate eliminating (a) protective group(s) temporarily introduced to protect other functional groups, and converting, where appropriate, the compound which has thus been obtained into its physiologically tolerated salts.

**2.** The process as claimed in claim 1, in which a compound of the formula I is prepared in which

L represents the radical of isoleucine, valine, threonine, serine, O-($C_1$-$C_9$)-alkylthreonine, O-($C_1$-$C_9$)-alkyl-serine, leucine, proline or of the $\omega$-($C_1$-$C_6$)-alkyl ester, preferably tert. butyl ester, of glutamic acid or aspartic acid;

N represents the radical of valine, isoleucine, leucine, phenylalanine, tryptophan, tyrosine which is optionally O-($C_1$-$C_6$)-alkylated, glutamine, asparagine, $\gamma$-($C_1$-$C_6$)-alkyl glutamate or $\beta$-($C_1$-$C_6$)-alkyl aspartate or $\epsilon$-acyl-lysine, and

$B^2$ denotes Arg, D-Arg, Lys or D-Lys,

and its physiologically tolerated salts.

**3.** The process as claimed in claim 1 or 2, in which is prepared a compound of the formula I in which p is 1, and R and $R^1$ together represent a peptide linkage, and its physiologically tolerated salts.

**4.** The process as claimed in one of claims 1 to 3, in which is prepared a compound in which R and $R^1$ do not together represent a peptide linkage, and its physiologically tolerated salts.

**5.** A process for the preparation of a pharmaceutical agent containing a compound of the formula I prepared as in one of claims 1-4, which comprises converting the latter, together with a vechicle and, where appropriate, other additives and auxiliaries, into a suitable form for administration.

**Revendications**

**Revendications pour les Etats contractants suivants : BE, CH, DE, FR, GB, IT, LI, LU, NL, SE**

**1.** Procédé pour la préparation d'un composé de formule I,

$R^N$-L-N-$B^2$-$R^C$ (I)

dans laquelle

$R^N$ représente un radical de formule II

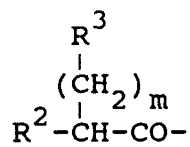

(II);

$R^2$ représente un atome d'hydrogène ou un radical de formule R-$(A)_n$-NH-;

$R^3$ représente un groupe amino, guanidino, alkyl($C_1$-$C_3$)-amino ou dialkyl($C_1$-$C_3$)-amino;

m représente un nombre entier allant de 1 à 6;

A représente un radical de formule -NH-$CR^4R^5$-CO-;

R représente un atome d'hydrogène ou un radical alcanoyle en $C_1$-$C_6$, aroyle en $C_7$-$C_{11}$ qui, sur le fragment aromatique, est éventuellement mono- ou disubstitué par un ou des substituants alkyle en $C_1$-$C_4$, alcoxy en $C_1$-$C_4$, alkyl- ($C_1$-$C_4$)-thio, halogène, carbamoyle, alcoxy($C_1$-$C_4$)-carbonyle et/ou sulfamoyle, ou monosubstitué par le groupe méthylènedioxy, un radical cycloalkyl($C_5$-$C_7$)-alcanoyle($C_1$-$C_3$) ou aryl($C_6$-$C_{14}$)-alcanoyle($C_1$-$C_3$), dans les radicaux R différents d'un atome d'hydrogène, un groupe -$CH_2$- étant éventuellement remplacé par -O- ou -S-;

n est 0 ou 1;

$R^4$ et $R^5$ sont identiques ou différents et représentent un atome d'hydrogène ou un groupe alkyle en $C_1$-$C_6$ ou aralcanoyle en $C_7$-$C_{11}$;

L représente Pro, D-Pro ou un radical de formule -NH-CH($R^6$)-CO-;

$R^6$ représente un radical alkyle en $C_1$-$C_6$ qui est éventuellement monosubstitué par un groupe hydroxy, alcoxy en $C_1$-$C_6$, alcoxy($C_1$-$C_6$)-carbonyle, carbamoyle ou R-NH, R étant tel que défini plus haut, mais ne pouvant pas être un atome d'hydrogène, ou un radical aralkyle en $C_7$-$C_{11}$ qui est éventuellement monosubstitué sur le fragment aromatique par un groupe alcoxy en $C_1$-$C_6$, ou le radical indol-3-ylméthyle;

N représente Pro, D-Pro ou un radical de formule -NH-CH($R^7$)-CO-;

$R^7$ représente un radical alkyle en $C_1$-$C_6$ qui est éventuellement monosubstitué par un groupe hydroxy, alcoxy en $C_1$-$C_6$, alcoxy($C_1$-$C_6$)-carbonyle, carbamoyle ou R-NH, R étant tel que défini plus haut, mais ne pouvant pas être un atome d'hydrogène, ou un radical aralkyle en $C_7$-$C_{11}$ qui est éventuellement monosubstitué sur le fragment aromatique par un groupe alcoxy en $C_1$-$C_6$, ou le radical indol-3-ylméthyle;

$B^2$ représente un radical de formule III

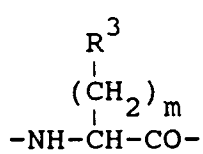

(III)

dans laquelle $R^3$ et m sont tels que définis plus haut;

$R^C$ représente un radical de formule -$NR^9$-CH($R^8$)-$(CO)_p$-$R^1$;

$R^8$ est défini comme $R^7$, des radicaux CH-, $CH_2$- ou $CH_3$- présents en position $\beta$ par rapport à -NH- étant éventuellement monohydroxylés; et

$R^9$ représente un atome d'hydrogène;

ou

$R^8$ et $R^9$ représentent ensemble -$(CH_2)_3$- ou -$(CH_2)_4$-;

p est 0 ou 1;

$R^1$ représente, lorsque p = 0, un atome d'hydrogène ou un groupe hydroxy ou alcoxy en $C_1$-$C_6$;

$R^1$ représente, lorsque p = 1, $OR^{10}$ ou $NR^{10}R^{11}$;

et

$R^{10}$ et $R^{11}$ sont identiques ou différents et représentent un atome d'hydrogène ou un groupe alkyle en $C_1$-$C_6$ ou aralkyle en $C_7$-$C_{11}$, ou $NR^{10}R^{11}$ représente le groupe pyrrolidino, pipéridino ou morpholino;

ou

P = 1,

R et $R^1$ représentent ensemble une liaison peptidique et les autres radicaux sont tels que définis plus haut,

et sels physiologiquement acceptables de celui-ci.

**2.** Composé de formule I selon la revendication 1, dans lequel

L représente le reste de l'isoleucine, de la valine, de la thréonine, de la sérine, d'une O-alkyl-($C_1$-$C_9$)-thréonine, d'une O-alkyl($C_1$-$C_9$)-sérine, de la leucine, de la proline ou d'un ester d'$\omega$-alkyle($C_1$-$C_6$), de préférence de tert-butyle, de l'acide glutamique ou de l'acide aspartique;

N représente le reste de la valine, de l'isoleucine, de la leucine, de la phénylalanine, du tryptophane, d'une tyrosine éventuellement O-alkylée en $C_1$-$C_6$, de la glutamine, de l'asparagine, d'un glutamate de $\gamma$-alkyle($C_1$-$C_6$) ou d'un aspartate de $\beta$-alkyle($C_1$-$C_6$), ou d'une $\epsilon$-acyl-lysine;

et

$B^2$ représente Arg, D-Arg, Lys ou D-Lys,

et sels physiologiquement acceptables de celui-ci.

**3.** Composé de formule I selon la revendication 1 ou 2, dans lequel p = 1 et R et $R^1$ représentent ensemble une liaison peptidique, et sels physiologiquement acceptables de celui-ci.

**4.** Composé de formule I selon la revendication 1 ou 2, dans lequel R et $R^1$ ne représentent pas ensemble une liaison peptidique, et sels physiologiquement acceptables de celui-ci.

**5.** Procédé pour la préparation d'un composé de formule 1 selon l'une des revendications 1 à 4, caractérisé en ce que l'on assemble un fragment à groupe carboxy terminal, ou un dérivé réactif, avec un fragment correspondant à groupe amino libre, éventuellement on élimine un(des) groupe(s) protecteur(s) temporairement introduit(s) pour la protection d'autres groupes fonctionnels, et éventuellement on convertit le composé ainsi obtenu en un de ses sels physiologiquement acceptable.

**6.** Composé de formule I selon l'une des revendications 1 à 4, pour utilisation en tant que médicament.

**7.** Composé de formule I selon l'une des revendications 1 à 4, pour utilisation en tant que médicament à effets diurétique et natriurétique.

**8.** Produit pharmaceutique contenant un composé de formule I selon l'une des revendications 1 à 4.

**9.** Procédé pour la fabrication d'un produit pharmaceutique selon la revendication 8, caractérisé en ce que l'on met sous une forme pharmaceutique appropriée un composé selon l'une des revendications 1 à 4, conjointement avec un véhicule et éventuellement d'autres additifs et adjuvants.

**Revendications pour les Etats contractants suivants : AT, ES, GR**

**1.** Procédé pour la préparation d'un composé de formule I,

$R^N$-L-N-$B^2$-$R^C$ (I)

dans laquelle

$R^N$ représente un radical de formule II

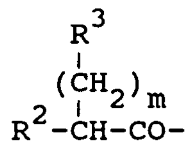 (II);

| | |
|---|---|
| $R^2$ | représente un atome d'hydrogène ou un radical de formule $R-(A)_n-NH-$; |
| $R^3$ | représente un groupe amino, guanidino, alkyl($C_1$-$C_3$)-amino ou dialkyl($C_1$-$C_3$)-amino; |
| m | représente un nombre entier allant de 1 à 6; |
| A | représente un radical de formule $-NH-CR^4R^5-CO-$; |
| R | représente un atome d'hydrogène ou un radical alcanoyle en $C_1$-$C_6$, aroyle en $C_7$-$C_{11}$ qui, sur le fragment aromatique est éventuellement mono- ou disubstitué par un ou des substituants alkyle en $C_1$-$C_4$, alcoxy en $C_1$-$C_4$, alkyl($C_1$-$C_4$)-thio, halogène, carbamoyle, alcoxy($C_1$-$C_4$)-carbonyle et/ou sulfamoyle, ou monosubstitué par le groupe méthylènedioxy, un radical cycloalkyl($C_5$-$C_7$)-alcanoyle($C_1$-$C_3$) ou aryl($C_6$-$C_{14}$)-alcanoyle($C_1$-$C_3$), dans les radicaux R différents d'un atome d'hydrogène, un groupe $-CH_2-$ étant éventuellement remplacé par -O- ou -S-; |
| n | est 0 ou 1; |
| $R^4$ et $R^5$ | sont identiques ou différents et représentent un atome d'hydrogène ou un groupe alkyle en $C_1$-$C_6$ ou aralcanoyle en $C_7$-$C_{11}$; |
| L | représente Pro, D-Pro, ou un radical de formule $-NH-CH(R^6)-CO-$; |
| $R^6$ | représente un radical alkyle en $C_1$-$C_6$ qui est éventuellement monosubstitué par un groupe hydroxy, alcoxy en $C_1$-$C_6$, alcoxy($C_1$-$C_6$)-carbonyle, carbamoyle ou R-NH, R étant tel que défini plus haut, mais ne pouvant pas être un atome d'hydrogène, ou un radical aralkyle en $C_7$-$C_{11}$ qui est éventuellement monosubstitué sur le fragment aromatique par un groupe alcoxy en $C_1$-$C_6$, ou le radical indol-3-ylméthyle; |
| N | représente Pro, D-Pro ou un radical de formule $-NH-CH(R^7)-CO-$; |
| $R^7$ | représente un radical alkyle en $C_1$-$C_6$ qui est éventuellement monosubstitué par un groupe hydroxy, alcoxy en $C_1$-$C_6$, alcoxy($C_1$-$C_6$)-carbonyle, carbamoyle ou R-NH, R étant tel que défini plus haut, mais ne pouvant pas être un atome d'hydrogène, ou un radical aralkyle en $C_7$-$C_{11}$ qui est éventuellement monosubstitué sur le fragment aromatique par un groupe alcoxy en $C_1$-$C_6$, ou le radical indol-3-ylméthyle; |
| $B^2$ | représente un radical de formule III |

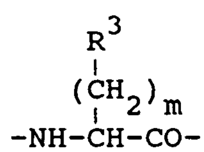 (III)

| | |
|---|---|
| | dans laquelle $R^3$ et m sont tels que définis plus haut; |
| $R^C$ | représente un radical de formule $-NR^9-CH(R^8)-(CO)_p-R^1$; |
| $R^8$ | est défini comme $R^7$, des radicaux CH-, $CH_2$- ou $CH_3$- présents en position $\beta$ par rapport à -NH- étant éventuellement monohydroxylés; et |
| $R^9$ | représente un atome d'hydrogène; |
| | ou |
| $R^8$ et $R^9$ | représentent ensemble $-(CH_2)_3-$ ou $-(CH_2)_4-$; |
| p | est 0 ou 1; |
| $R^1$ | représente, lorsque p = 0, un atome d'hydrogène ou un groupe hydroxy ou alcoxy en $C_1$-$C_6$; |
| $R^1$ | représente, lorsque p = 1, $OR^{10}$ ou $NR^{10}R^{11}$; |
| | et |
| $R^{10}$ et $R^{11}$ | sont identiques ou différents et représentent un atome d'hydrogène ou un groupe alkyle en $C_1$-$C_6$ ou aralkyle en $C_7$-$C_{11}$, ou $NR^{10}R^{11}$ représente le groupe pyrrolidino, pipéridino ou morpholino; |
| | ou |
| P | = 1, |
| R et $R^1$ | représentent ensemble une liaison peptidique et les autres radicaux sont tels que |

définis plus haut,

ainsi que de ses sels physiologiquement acceptables, caractérisé en ce que l'on assemble un fragment à groupe carboxy terminal, ou un dérivé réactif, avec un fragment correspondant à groupe amino libre, éventuellement on élimine un(des) groupe(s) protecteur(s) temporairement introduit(s) pour la protection d'autres groupes fonctionnels, et éventuellement on convertit le composé ainsi obtenu en un de ses sels physiologiquement acceptable.

**2.** Procédé selon la revendication 1, caractérisé en ce que l'on prépare un composé de formule I selon la revendication 1, dans lequel

L représente le reste de l'isoleucine, de la valine, de la thréonine, de la sérine, d'une O-alkyl-($C_1$-$C_9$)-thréonine, d'une O-alkyl($C_1$-$C_9$)-sérine, de la leucine, de la proline ou d'un ester d'$\omega$-alkyle($C_1$-$C_6$), de préférence de tert-butyle, de l'acide glutamique ou de l'acide aspartique;

N représente le reste de la valine, de l'isoleucine, de la leucine, de la phénylalanine, du tryptophane, d'une tyrosine éventuellement O-alkylée en $C_1$-$C_6$, de la glutamine, de l'asparagine, d'un glutamate de $\gamma$-alkyle($C_1$-$C_6$) ou d'un aspartate de $\beta$-alkyle($C_1$-$C_6$), ou d'une $\epsilon$-acyl-lysine;

et

$B^2$ représente Arg, D-Arg, Lys ou D-Lys,

ainsi que ses sels physiologiquement acceptables.

**3.** Procédé selon la revendication 1 ou 2, caractérisé en ce que l'on prépare un composé de formule I, dans lequel p = 1 et R et $R^1$ représentent ensemble une liaison peptidique, ainsi que ses sels physiologiquement acceptables.

**4.** Procédé selon l'une des revendications 1 à 3, caractérisé en ce que l'on prépare un composé de formule I dans lequel R et $R^1$ ne représentent pas ensemble une liaison peptidique, ainsi que ses sels physiologiquement acceptables.

**5.** Procédé pour la fabrication d'un produit pharmaceutique contenant un composé de formule 1 préparé selon l'une des revendications 1 à 4, caractérisé en ce que l'on met sous une forme pharmaceutique appropriée ce composé, conjointement avec un véhicule et éventuellement d'autres adjuvants et additifs.